(19) Europäisches Patentamt
European Patent Office
Office européen des brevets

(11) **EP 4 442 194 B1**

(12) **EUROPEAN PATENT SPECIFICATION**

(45) Date of publication and mention
of the grant of the patent:
**10.12.2025 Bulletin 2025/50**

(21) Application number: **23870269.0**

(22) Date of filing: **08.09.2023**

(51) International Patent Classification (IPC):
*A61B 5/00* (2006.01)       *A61B 5/366* (2021.01)
*A61B 5/352* (2021.01)      *A61B 5/318* (2021.01)
*A61B 5/024* (2006.01)     *A61B 5/346* (2021.01)

(52) Cooperative Patent Classification (CPC):
**A61B 5/00; A61B 5/318; A61B 5/346;
A61B 5/7203; A61B 5/7225;** A61B 5/02438;
A61B 5/725

(86) International application number:
**PCT/CN2023/117833**

(87) International publication number:
**WO 2024/067030 (04.04.2024 Gazette 2024/14)**

(54) **SIGNAL DENOISING METHOD AND ELECTRONIC DEVICE**

SIGNALENTRAUSCHUNGSVERFAHREN UND ELEKTRONISCHE VORRICHTUNG

PROCÉDÉ DE DÉBRUITAGE DE SIGNAL ET DISPOSITIF ÉLECTRONIQUE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **29.09.2022 CN 202211202646**

(43) Date of publication of application:
**09.10.2024 Bulletin 2024/41**

(73) Proprietor: **Honor Device Co., Ltd.
Shenzhen, Guangdong 518040 (CN)**

(72) Inventor: **YIN, Haibo
Shenzhen, Guangdong 518040 (CN)**

(74) Representative: **Gill Jennings & Every LLP
The Broadgate Tower
20 Primrose Street
London EC2A 2ES (GB)**

(56) References cited:
CN-A- 106 889 984       CN-A- 113 040 784
CN-A- 114 521 899       CN-A- 114 938 951
US-A1- 2007 260 151     US-B1- 6 599 242

• GAUTAM ALKA ET AL: "ECG Signal De-noising
with Signal Averaging and Filtering Algorithm",
11 November 2008 (2008-11-11), Los Alamitos,
CA, USA, pages 409 - 415, XP093229580, ISBN:
978-0-7695-3407-7, Retrieved from the Internet
<URL:https://ieeexplore.ieee.org/stampPDF/
getPDF.jsp?tp=&arnumber=4682061&
ref=aHR0cHM6Ly9pZWVleHBsb3JlLmllZ
WUub3JnL2RvY3VtZW50LzQ2ODIwNjE=>
[retrieved on 20241129], DOI: 10.1109/
ICCIT.2008.393

## Description

This application claims priority to Chinese Patent Application No. 202211202646.9, filed with the China National Intellectual Property Administration on September 29, 2022 and entitled "METHOD FOR REMOVING NOISE FROM SIGNAL, AND ELECTRONIC DEVICE".

## TECHNICAL FIELD

This application relates to the field of vital sign identification and processing technologies, and in particular, relates to a method for removing noise from a signal, and an electronic device.

## BACKGROUND

An electrocardiography signal is acquired generally by an electrode attached to a skin surface. Since the electrocardiography signal on a skin is weak and susceptible to noise interference, the collected electrocardiography signal contains much noise. This reduces accuracy and reliability of electrocardiography diagnosis. Particularly, electrocardiography of a non-stationary user acquired by a wearable electrocardiography device contains plenty of noise.

Noise such as electromyography noise and motion noise in the electrocardiography signal is found in a full frequency band of the electrocardiography signal. Effects of such noise are small but difficult to eradicate. Effects of such noise in a stationary segment of the signal are more apparent. When such noise in the electrocardiography signal is removed through a conventional method for removing noise, wave peaks of a P wave, a T wave and an R wave in the electrocardiography signal are likely to be disturbed. If the P wave, T wave and R wave serving as critical features in the electrocardiography signal are effected, the electrocardiography signal will lose a reference value.

Therefore, how to remove noise from an electrocardiography signal on the premise of guaranteeing a reference value of the electrocardiography signal is an urgent problem to be resolved. Patent application CN113040784A describes an electromyography noise filtering method for electrocardiosignals. Firstly, wavelet-Wiener filtering is adopted to carry out mild filtering on the whole signals, details of high-frequency components are fully reserved while electromyography noise of the electrocardiosignal high-frequency components is filtered out, and then moving average filtering is adopted to further filter out residual noise; after wavelet-Wiener filtering is carried out on the whole signals, additional processing is carried out on low-frequency components, a Q point and an S point are positioned firstly, the low-frequency components between two heart beats are intercepted, and only the low-frequency components are filtered by moving average to further filter out the residual electromyography noise of the low-frequency components of the electrocardiosignals; the severity of electromyography noise in the signals is evaluated before the two steps of filtering, so that parameters of a filter are adaptively set; before moving average filtering is carried out on the low-frequency components, points with half of the moving average filtering order are spliced on the two sides respectively. According to the method, the noise in the electrocardiosignal can be reduced with minimum distortion, and the method can be used as an effective tool for denoising the electrocardiosignal.

## SUMMARY

An objective of this application is to provide a method for removing noise from a signal, and an electronic device. According to the method, by virtue of a continuous abrupt change performance of a wave peak having reference significance in an electrocardiography signal, whether a signal point is a point on a signal wave having reference significance can be identified during noise removal, and whether to remove noise from the signal point can be determined. The method is implemented, so that noise can be effectively removed from the electrocardiography signal on the premise that a reference value of the electrocardiography signal is guaranteed.

The above and other objectives are achieved by using features in independent claims. Further implementations are embodied in dependent claims, description and accompanying drawings.

In a first aspect, this application provides a method for removing noise from a signal. The method includes: determining a first feature sequence corresponding to a target sampling point, where the first feature sequence corresponding to the target sampling point includes j elements, the j elements correspond to, in a one-to-one manner, j sampling points in a signal to undergo noise removal, an element corresponding to any sampling point of the j sampling points represent a sum of rising or falling ranges of all sampling points between the any sampling point and a first sampling point, a falling or rising range of the any sampling point of the j sampling points is equal to a difference between an amplitude of the any sampling point and an amplitude of a sampling point after the any sampling point, and the first sampling point is a sampling point that is located before the any sampling point, has an opposite change tendency relative to the any sampling point, and is closest to the any sampling point; and averaging an amplitude of the target sampling point and amplitudes of m sampling points around the target sampling point, and obtaining a target numerical value in a case that numerical values in the first feature sequence corresponding to the target sampling point are all less than a cumulative variation threshold corresponding to the target sampling point, and updating the amplitude of the target sampling point to the target numerical value, where the cumulative variation threshold corresponding to the target sampling point is determined by using the first feature sequence corresponding to the target sampling point and v first feature sequences corresponding to v sampling points before the target sampling point.

In the method, the target sampling point is a sampling point in a signal to undergo noise removal. The signal to undergo noise removal may be a signal directly acquired by an electronic device, or a signal acquired by another device and transmitted to the electronic device. Then, the electronic device may further transmit a signal obtained by removing noise from the signal with noise removal to the another device.

Specifically, the signal to undergo noise removal may be an electrocardiography signal. The electrocardiography signal may be a bioelectricity signal of a biological body surface extracted by the electronic device by using an electrode. The acquired electrocardiography signal is composed of a plurality of sampling points ordered according to sampling time. In the electrocardiography signal, a value of the sampling point is an intensity or energy value of a bioelectricity signal of the body surface during acquisition. The plurality of sampling points are arranged in order of sampling time to form electrocardiography having a specific fluctuation degree. A QRS wave in the electrocardiography signal is generally the largest, most obvious and sharpest wave. This feature is mathematically referred to as singularity or abrupt change performance of the QRS wave and shown as an abrupt change in slope. That is, an amplitude difference between two adjacent sampling points on a QRS complex in the electrocardiography signal is extremely great compared with that of a stationary-segment signal in the electrocardiography signal. Thus, an absolute value of slope between two adjacent sampling points in the QRS complex is extremely great.

The expression "has an opposite change tendency" means that an amplitude of a sampling point before the first sampling point is greater than an amplitude of the first sampling point, but an amplitude of a sampling point after the first sampling point is less than the amplitude of the first sampling point; or means that the amplitude of the sampling point before the first sampling point is less than the amplitude of the first sampling point, but the amplitude of the sampling point after the first sampling point is greater than the amplitude of the first sampling point. Specifically, on an image, the first sampling point may be a sampling point located on a wave peak or a wave trough in a signal to be detected.

In addition, the expression "sum of rising or falling ranges" means either a sum of rising ranges or a sum of falling ranges. That is, as observed from only one change tendency, whether a sufficient number of continuous sampling points of which amplitudes keep sharply rising exist in the j sampling points or whether a sufficient number of continuous sampling points of which amplitudes keep sharply falling exist in the j sampling points is determined.

Further, j may be a positive integer greater than 1. In an optional implementation, the j may be determined according to a sampling frequency of the signal to undergo noise removal. Specifically, j may be equal to thirty percent of a sampling frequency of the signal to undergo noise removal. For example, if the sampling frequency of the signal with noise removal is 500, j may be $500 \times 0.3 = 150$.

It should be understood that one wave can be regarded as two parts according to a change tendency, that is, a rising part (in which the amplitude of the sampling point is increasingly great) and a falling part (in which the amplitude of the sampling point is increasingly great). The wave peak rises and then falls. The wave trough falls and then rises. The amplitude of the sampling point in the QRS complex may change in a fixed tendency for longer time than that of the stationary-segment signal. For example, in a QS segment of the QRS complex, the amplitude of the signal persistently rises and then persistently falls. That is, in the electrocardiography signal, in a case that a segment of sub-signal is selected from the electrocardiography signal in a time window (for example, a time window including 30 sampling points) having a fixed length, if the segment of sub-signal is a signal completely located in the QRS complex, an amplitude of the segment of sub-signal is extremely likely to sharply rise or sharply fall. If the segment of sub-signal is a signal located in a stationary-segment signal, a change tendency of the amplitude of the segment of signal is extremely likely to repeatedly change. For example, the amplitude rises, falls, rises, falls..., and repeatedly changes in this way. Moreover, a change range (which is embodied in the image as whether a wave is sharp enough) of the segment of sub-signal may be extremely small.

Thus, the electronic device can carry out reference with one change tendency as a dimension, and can determine whether a signal is a signal on the QRS complex by viewing persistence and an abrupt change performance of a segment of signal determined by using a sampling point in a change tendency, that is, whether a segment of continuous sampling points exists in the segment of signal, whether a number of the sampling points is sufficient and amplitudes of the sampling points keep sharply rising or falling, and further determine whether the sampling point is a point on or around the QRS complex. Based on this, in the method, the electronic device can construct, based on a change tendency and range of an amplitude of each sampling point and sampling points around the sampling point, a feature sequence reflecting whether the point is a sampling point on the QRS complex. The feature sequence may be referred to as a first feature sequence corresponding to the point.

It should be understood that in a process that the electronic device removes noise from the signal to undergo noise removal, the electronic device determines a sampling point of the signal to undergo noise removal as the target sampling point, and then determines the first feature sequence corresponding to the target sampling point through the method in the foregoing description. After processing the amplitude of the target sampling point, the electronic device takes the sampling point after the target sampling point as a new target sampling point and acquires a first feature sequence corresponding to the new target sampling point through the same method. It is not difficult to understand that when the target sampling point changes, a first time window corresponding to the target sampling point will also move backwards (generally by one sampling point) accordingly. That is, the electronic device takes each sampling point before the target sampling point as a target sampling point and acquires first feature sequences corresponding to these sampling points.

In the method, after determining the first feature sequence corresponding to the target sampling point, the electronic device determines a threshold, that is, the cumulative variation threshold corresponding to the target sampling point, based on the first feature sequence corresponding to the target sampling point and the first feature sequences corresponding to the v sampling points before the target sampling point. The cumulative variation threshold represents a distribution range of values of most elements in a sequence obtained by splicing the first feature sequence corresponding to the target sampling point and the v first feature sequences corresponding to the v sampling points before the target sampling point. If a value of an element in the sequence is greater than or equal to the cumulative variation threshold, it indicates that the value of the element is significantly greater than values of the other elements in the sequence. That is, the element is an element on which an abrupt change occurs, which exactly conforms to the abrupt change performance of the QRS complex in the electrocardiography signal. Therefore, in the method, if the numerical values of the elements in the first feature sequence corresponding to the target sampling point are all less than the cumulative variation threshold corresponding to the target sampling point, it indicates that the target sampling point is extremely likely to be a sampling point in a stationary-segment signal located in the signal to undergo noise removal. The electronic device may average an amplitude of the target sampling point and amplitudes of m sampling points around the target sampling point through an averaging filter method, obtain a target numerical value, and update the amplitude of the target sampling point to the target numerical value. Therefore, noise is removed from the target sampling point. Accordingly, if a numerical value of an element in the first feature sequence corresponding to the target sampling point is greater than or equal to the cumulative variation threshold corresponding to the target sampling point, it indicates that the target sampling point is extremely likely to be a sampling point located in the QRS complex in the signal to undergo noise removal. To protect the features of the QRS complex, the electronic device does not change the amplitude of the target sampling point.

According to the method, by virtue of a continuous abrupt change performance of a wave peak having reference significance in an electrocardiography signal, whether a sampling point is a point on a signal wave having reference significance can be identified during noise removal, and whether to remove noise from the sampling point is determined. The method is implemented, so that noise can be effectively removed from the electrocardiography signal on the premise that a reference value of the electrocardiography signal is guaranteed.

In combination with the first aspect, in a possible implementation, before the averaging an amplitude of the target sampling point and amplitudes of m sampling points around the target sampling point, and obtaining a target numerical value, the method further includes: determining a first feature value corresponding to the target sampling point, where the first feature value corresponding to the target sampling point represents an average of absolute values of amplitude differences between every two adjacent sampling points in a time window including the target sampling point and including (k+1) sampling points; and the averaging an amplitude of the target sampling point and amplitudes of m sampling points around the target sampling point, and obtaining a target numerical value includes: averaging the amplitude of the target sampling point and the amplitudes of the m sampling points around the target sampling point, and obtaining the target numerical value in a case that the numerical values in the first feature sequence corresponding to the target sampling point are all less than the cumulative variation threshold corresponding to the target sampling point, and the first feature value corresponding to the target sampling point is less than an average variation threshold. The average variation threshold corresponding to the target sampling point is determined by using the first feature value corresponding to the target sampling point and first feature values corresponding to i sampling points. In the signal to undergo noise removal, the i sampling points are i sampling points before the target sampling point.

It should be understood that in cases of some abnormal electrocardiography signals, a direction of an R wave in the QRS complex in the signal may be reversed. For example, R waves in QRS complexes in electrocardiography signals of some people suffering from heart diseases may be inverted due to myocardial ischemia of the patients. In this case, rising and falling tendencies of the sampling point in the R wave are reversed. Change tendencies between wave peaks and wave troughs of the R wave and PR and ST segments are reversed. In this case, if a position of the target sampling point is analyzed only by constructing the first feature sequence corresponding to the target sampling point, an erroneous conclusion is likely to be acquired. Moreover, the amplitude of the target sampling point is extremely likely to be processed through an improper method in a noise removal process. However, the first feature value corresponding to the target sampling point focuses on only a change range of the amplitude of the sampling point and does not focus on a change tendency of the amplitude of the sampling point. In the implementation, a region where the target sampling point is located is determined by using the first feature value corresponding to the target sampling point and the first feature sequence corresponding to the target sampling point such that determination of a position of a sampling point on an abnormal electrocardiography signal can be extremely likely to be reduced, whether the target signal point is a point on the QRS complex is determined, and noise is removed from the stationary-segment signal in the signal on the premise that features of the Q wave and the S wave are retained. In addition, by using the first feature value corresponding to the target sampling point, the electronic device can more quickly and accurately determine whether a normal electrocardiography signal is a sampling point on the R wave.

In combination with the first aspect, in a possible implementation, the method further includes: keeping the amplitude of the target sampling point unchanged in a case that at least one numerical value in the first feature sequence corresponding to the target sampling point is greater than or equal to the cumulative variation threshold corresponding to the target sampling point; and/or keeping the amplitude of the target sampling point unchanged in a case that the first feature value corresponding to the target sampling point is greater than or equal to the average variation threshold.

It should be understood that in a case that at least one numerical value in the first feature sequence corresponding to the target sampling point is greater than or equal to the cumulative variation threshold corresponding to the target sampling point, the target sampling point is extremely likely to be a point on the QRS complex. In addition, in a case that the first feature value corresponding to the target sampling point is greater than or equal to the average variation threshold, the target sampling point is likely to be a point on the QRS complex and is extremely likely to be a point on the R wave. Therefore, in the implementation, to protect a reference value of a key wave in a signal after noise removal, when determining that the target sampling point is a point on the QRS complex, the electronic device keeps the amplitude of the target sampling point unchanged.

In combination with the first aspect, in a possible implementation, before the determining a first feature sequence corresponding to a target sampling point, the method further includes: high-pass-filtering and low-pass-filtering an initial signal, and obtaining the signal to undergo noise removal. The initial signal is an electrical signal that represents a heart rhythm of a user and is acquired by an electronic device.

In the implementation, the electronic device further high-pass-filters and low-pass-filters an initial signal, and obtains the signal to undergo noise removal. The initial signal is an electrical signal that represents a heart rhythm of a user and is acquired by an electronic device. A high-pass filter and a low-pass filter used by the electronic device may be multi-order filters. These filters can filter out higher and lower frequency bands, especially those frequency bands that may include artifacts, of an initial signal acquired by the electronic device. Subsequent analysis is facilitated, a frequency range of a signal to be analyzed is retained, and processing efficiency of a subsequent processing process is improved.

Specifically, in a high-frequency band, the electronic device may use a low-pass filter having a cut-off frequency lower than an alternating-current electrical frequency (50 Hz or 60 Hz) to avoid power frequency disturbance. In a low-frequency band, that is, a frequency band lower than 1 Hz, the electronic device may use a high-pass filter that retains a highest frequency band of a signal of interest.

In combination with the first aspect, in a possible implementation, in the time window including the target sampling point and including (k+1) sampling points, the target sampling point is a last sampling point in the time window including the (k+1) sampling points; or in the time window including the target sampling point and including the (k+1) sampling points, at least one sampling point exists before the target sampling point and at least one sampling point exists after the target sampling point.

In the implementation, after currently acquiring a latest sampling point signal, the electronic device may acquire k sampling points before the sampling point, and compute an average of absolute values of amplitude differences between every two adjacent sampling points of the (k+1) sampling points ending with the sampling point as the first feature value corresponding to the sampling point. In this way, the electronic device can process the just-acquired sampling point without delay, and more efficiently process the target sampling point. Alternatively, after currently acquiring a signal of a latest one sampling point, the electronic device may not immediately process the sampling point, but acquires at least one sampling point before the sampling point and at least one sampling point before the sampling point, which are k sampling points in total, with the sampling point as a base point after continuing acquiring signals of several sampling points after the sampling point, determines the k sampling points and the sampling point as the (k+1) sampling points, and computes the average of the absolute values of the amplitude differences between every two adjacent sampling points of the (k+1) sampling points as the first feature value corresponding to the sampling point. In this way, although the electronic device cannot process the just-acquired sampling point, by taking the target sampling point as the sampling point at a middle position of the (k+1) sampling points, the computed first feature value corresponding to the target sampling point can more realistically reflect the change tendency of the target sampling point, and whether the target sampling point is a point on the QRS complex can be more accurately determined.

In combination with the first aspect, in a possible implementation, the determining a first feature sequence corresponding to a target sampling point includes: taking the target sampling point as a start point, and determining a first time window including (j+1) sampling points; computing an amplitude difference between each sampling point except for a last sampling point in the first time window and a sampling point after the sampling point in sequence, and obtaining j differences; resetting numerical values less than 0 in the j differences as 0, and obtaining a first sequence corresponding to the target sampling point; and carrying out a forward accumulation and reconstruction operation on elements in the first sequence corresponding to the target sampling point in sequence, obtaining the j elements, and taking the j elements as the first feature sequence corresponding to the target sampling point. The accumulation and reconstruction operation includes: keeping a value of an element unchanged in a case that the value of the element is 0; and accumulating, in a case that the value of the element is not zero, the value of the element and a value of a previous element until an element having a value of 0 is encountered, and taking a value obtained through forward accumulation as a value after element reconstruction.

Specifically, in an optional implementation, the above electronic device may determine the first feature sequence corresponding to the target sampling point by using the above target sampling point through the following method:

(1) Assuming that $x_t$ is the target sampling point, setting a time window as j+1, acquiring an amplitude of each sampling point within the window, and obtaining sequence $X_B$, such that $X_B = [x_t, x_{t+1}..., x_{t+j}]$. (2) Computing a backward difference of adjacent elements in $X_B$, and obtaining sequence $M_{B1}$, such that $M_{B1} = [x_t - x_{t+1},..., x_{t+j-1} - x_{t+j}]$. (3) Setting data less than zero in sequence $M_{B1}$ as zero, that is, $M_{B1}[M_{B1} < 0] = 0$, and obtaining sequence $M_{B2}$. (4) Carrying out a forward accumulation and reconstruction operation on each original element in sequence $M_{B2}$, and obtaining first feature sequence $M_B$ corresponding to sampling point $x_t$. Specifically, if an element in sequence $M_{B2}$ is 0, the position is still 0 after reconstruction. If the position is not zero, forward accumulation is carried out until an element that is 0 is encountered. A value obtained through the forward accumulation is a value of the position after reconstruction.

Through the implementation, a cumulative change tendency of subsequent j sampling points around the target sampling point is quantified in the first feature sequence corresponding to the target sampling point through amplitude subtraction, negative value zero setting, and forward accumulation and reconstruction. A plurality of identical elements exist in the first feature sequence corresponding to two continuous sampling points such that the electronic device can favorably analyze data features of the target sampling point, so as to determine a region where the target sampling point is located.

In combination with the first aspect, in a possible implementation, the determining a first feature value corresponding to the target sampling point includes: determining a second time window including (k+1) sampling points, where in the second time window, n sampling points exist before the target sampling point, and (k-n) sampling points exist after the target sampling point; and computing an absolute value of a difference between each sampling point and a sampling point before the sampling point in the second time window in sequence, obtaining absolute values of k differences, and taking an average of the absolute values of the k differences as the first feature value corresponding to the target sampling point.

Specifically, in the implementation, the above electronic device may determine the first feature value corresponding to the target sampling point by using the above target sampling point through the following method: (1) Assuming that $x_t$ is a target sampling point, setting a time window as k+1, obtaining an amplitude of each sampling point within the window, and obtaining sequence $X_A$, such that $X_A = [x_{t-a},..., x_{t-1}, x_t, x_{t+1},..., x_{t+b}]$, and k=a+b. (2) Computing average $m^A$ of absolute values of differences of sequence $X_A$, such that $m^A$ is the first feature value corresponding to target sampling point

$x_t$, and $m^A = \dfrac{\sum_{q=t-k}^{q=t+k}(|x_q - x_{q-1}|)}{2k+1}$ .

That is, the electronic device may determine a second time window including (k+1) sampling points based on the above target sampling point. In the second time window, n sampling points exist before the target sampling point, and (k-n) sampling points exist after the target sampling point. Then, the electronic device may compute an absolute value of a difference between each sampling point and a sampling point before the sampling point in the second time window in sequence, obtain absolute values of k differences, and take an average of the absolute values of the k differences as the first feature value corresponding to the target sampling point.

It should be understood that in the implementation, unlike a cumulative variation degree, represented by the first feature sequence, of a sampling point in a single change tendency, since the first feature value corresponding to the target sampling point is obtained based on the absolute value of the amplitude difference between two sampling points, a change range between the two sampling points is a number greater than 0 no matter how a change tendency of the (k+1) sampling points changes. That is, no matter whether an amplitude of a sampling point in the time window including (k+1) sampling points continuously rises, continuously falls, alternately rises or alternately falls, the first feature value corresponding to the target sampling point is an average of total slope between every two adjacent sampling points of the (k+1) sampling points. Then, in cases of sampling points on the QRS complex, especially on the R wave, slope (that is, an abrupt change degree) between each of points around these sampling points and a sampling point before the point and a sampling point after the point is extremely great, which means that the electronic device can easily determine whether the target sampling point is a point on the QRS wave, especially on the R wave, based on the first feature value corresponding to the target sampling point, so as to determine whether noise is required to be removed from the target sampling point.

In combination with the first aspect, in a possible implementation, after the determining a first feature sequence corresponding to a target sampling point, the method further includes: splicing the first feature sequence corresponding to the target sampling point and the v first feature sequences corresponding to the v sampling points, obtaining a second sequence, determining an outlier corresponding to the second sequence through a quartile method, and determining the outlier corresponding to the second sequence as the cumulative variation threshold corresponding to the target sampling point.

In the implementation, the above cumulative variation threshold may be determined through a quartile method. Since an indication giving a center, spread and a shape of data distribution has robustness and scientificity, by determining the cumulative variation threshold corresponding to the target sampling point through the quartile method, whether an element of which a numerical value sharply changes exists in the first feature sequence corresponding to the target sampling point can be accurately reflected to some extent, and further whether the target sampling point is a point on the QRS complex is reflected.

In combination with the first aspect, in a possible implementation, after the determining a first feature value corresponding to a target sampling point, the method further includes: constructing a third sequence by using the first feature value corresponding to the target sampling point and i first feature values corresponding to the i sampling points, determining an outlier corresponding to the third sequence through the quartile method, and determining the outlier corresponding to the third sequence as the average variation threshold corresponding to the target sampling point.

Specifically, $C_B = k1 * percentile(M_{BH}, 75) - k2 * percentile(M_{BH}, 25)$, where
"*" represents multiplication operation, $C_B$ is the above cumulative variation threshold, $M_{BH} = [M_{B-v}, M_{B-v+1}, ... , M_B]$, $M_B$ is the first feature sequence corresponding to the above target sampling point, $M_{B-1}$ is a first feature sequence corresponding to a sampling point before the above target sampling point, and so on.

Further, $M_{BH}$, is a sequence obtained by unfolding, splicing and constructing a plurality of sequences of $M_{B-v}$, $M_{B-v+1}$, ... , $M_B$, etc., that is, the above second sequence. Further, $percentile(M_{BH}, 75)$ represents a seventy-fifth quantile (upper quartile) of sequence $M_{BH}$, and $percentile(M_{BH}, 25)$ is a twenty-fifth quantile (lower quartile) of sequence $M_{BH}$. Values of $k1$ and $k2$ are $k1=4$ and $k2=3$, or $k1=2.5$ and $k2=1.5$. In cases of $k1=4$ and $k2=3$, $C_B$ is an extreme outlier corresponding to the second sequence. In cases of $k1=2.5$ and $k2=1.5$, $C_B$ is a moderate outlier corresponding to the second sequence. It is not difficult to see that the electronic device adaptively updates the above cumulative variation threshold as the target sampling point is replaced.

Similarly, in the implementation, by determining the average variation threshold corresponding to the target sampling point through the quartile method, whether the target sampling point is an element of which a numerical value sharply changes in the third sequence can be accurately reflected to a specific extent, and further whether the target sampling point is a point on the QRS complex (especially on the R wave) is reflected.

Specifically, the average variation threshold corresponding to the target sampling point may be expressed as $C_A = k1 * percentile(M_{AH}, 75) - k2 * percentile(M_{AH}, 25)$, where
"*" represents multiplication operation, $C_A$ is the cumulative variation threshold,

$$M_{AH} = [m_{t-i}^A, m_{t-i+1}^A, m_{t-i+2}^A, ..., m_t^A]$$ , $M_A$ is the first feature value corresponding to the target sampling point, $M_{A-1}$ is a first feature value corresponding to a sampling point before the above target sampling point, and so on. Further, $M_{AH}$ is the above third sequence. Further, $percentile(M_{AH}, 75)$ represents a seventy-fifth quantile (upper quartile) of sequence $M_{AH}$, and $percentile(M_{AH}, 25)$ is a twenty-fifth quantile (lower quartile) of sequence $M_{BAH}$. Values of $k1$ and $k2$ are $k1=4$ and $k2=3$, or $k1=2.5$ and $k2=1.5$. In cases of $k1=4$ and $k2=3$, $C_A$ is an extreme outlier corresponding to the third sequence. In cases of $k1=2.5$ and $k2=1.5$, $C_A$ is a moderate outlier corresponding to the third sequence. It is not difficult to see that the electronic device can also adaptively update the above average variation threshold as the target sampling point is replaced.

In combination with the first aspect, in a possible implementation, the cumulative variation threshold corresponding to the target sampling point is an extreme outlier corresponding to the second sequence determined through the quartile method, and/or the average variation threshold corresponding to the target sampling point is an extreme outlier corresponding to the third sequence determined through the quartile method.

In combination with the features of the quartile method, in the implementation, when the signal to undergo noise removal is a signal such as an electrocardiography signal having a new signal segment having an extremely-great amplitude sharp change degree, the electronic device may determine the cumulative variation threshold corresponding to the target sampling point as the extreme outlier corresponding to the second sequence, and/or determine the average variation threshold corresponding to the target sampling point as the extreme outlier corresponding to the third sequence.

In a second aspect, an embodiment of this application provides an electronic device. The electronic device includes: one or more processors and a memory. The memory is coupled to the one or more processors. The memory is configured to store computer program code. The computer program code includes computer instructions. The one or more processors invoke the computer instructions to enable the electronic device to perform the method as the first aspect or any possible implementation in the first aspect.

In a third aspect, a chip system is provided. The chip system is applied to an electronic device. The chip system includes one or more processors. The processor is configured to invoke computer instructions to enable the electronic device to perform the method as the first aspect or any possible implementation in the first aspect.

In a fourth aspect, a computer-readable storage medium is provided. The computer-readable storage includes instructions. The above instructions enable the above electronic device to perform the method as the first aspect or any possible implementation in the first aspect when running on an electronic device.

## BRIEF DESCRIPTION OF DRAWINGS

FIG. 1 is a waveform graph of an electrocardiography (EGG) signal according to an embodiment of this application;

FIG. 2 is a broken line graph representing a change degree of data in a sequence of number according to an embodiment of this application;

FIG. 3 is a schematic description diagram of a noise removal result of an electrocardiography signal according to an embodiment of this application;

FIG. 4 is a schematic diagram of appearance of an electronic device according to an embodiment of this application;

FIG. 5 is a schematic diagram of a structure of an electronic device according to an embodiment of this application;

FIG. 6 is a flow diagram of a method for removing noise from a signal according to an embodiment of this application;

FIG. 7 is a schematic diagram of a change tendency of an amplitude of a sub-signal in a middle segment of a signal to undergo noise removal according to an embodiment of this application;

FIG. 8 is a schematic diagram of a process of acquiring a first feature sequence corresponding to a target sampling point based on the target sampling point according to an embodiment of this application;

FIG. 9 is a schematic diagram of comparison between an image of an initial signal and an image of a first feature sequence according to an embodiment of this application;

FIG. 10 is a flow diagram of a method for removing noise from a signal according to an embodiment of this application; and

FIG. 11 is a schematic diagram of comparison between an image of an initial signal and an image of a first feature value according to an embodiment of this application.

## DESCRIPTION OF EMBODIMENTS

Terms used in the following embodiments of this application are only intended to describe particular embodiments, and are not intended to limit this application. As used in the description and claims of this application, a singular expression form, "one", "a", "an", "the", "the above", "mentioned above", "this", "these" and "such a", is intended to further include a plural expression form, unless clearly indicated to the contrary in the context. It should be further understood that the term "and/or" used in this application indicates and includes any or all possible combinations of one or more listed items.

Since embodiments of this application relate to application of a neural network, to facilitate understanding, related terms involved in embodiments of this application will be first introduced below.

(1) Complex and stationary-segment signal of electrocardiography signal

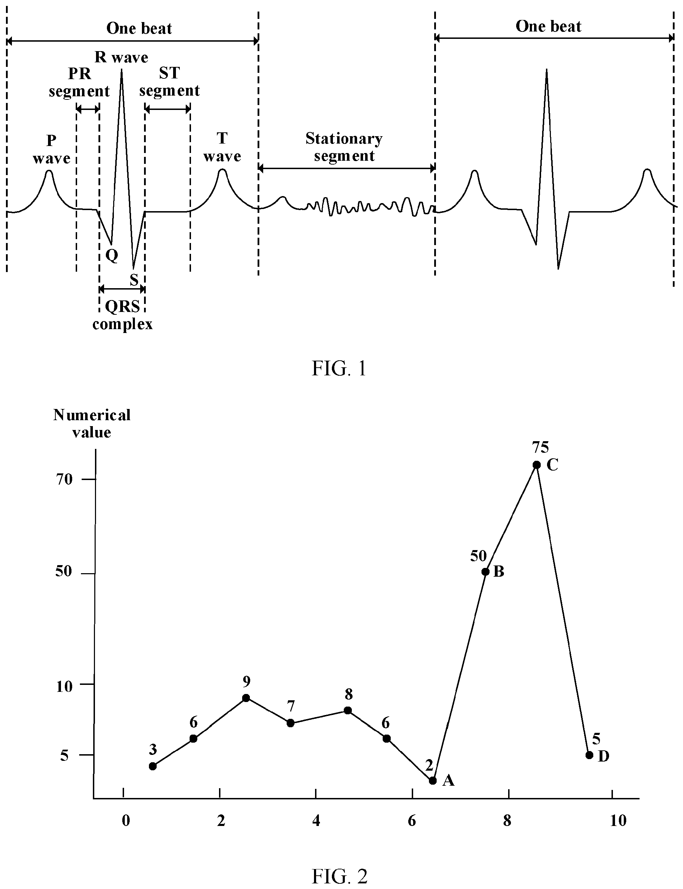

Electrocardiography (electrocardiography, ECG or EKG), also referred to as an electrocardiography signal, records bioelectricity signals generated during systole and diastole of a heart. Every time the heart completes a complete electrical activity, the activity corresponds to an ECG waveform shown in FIG. 1. The waveform includes a P wave, a QRS complex (including a Q wave, an R wave and an S wave), and a T wave. A first waveform deviating from a baseline in a forward direction on electrocardiography is a P wave, and the second wave band is a QRS complex. The QRS complex is composed of a series of 3 deviations and reflects electric currents associated with left and right ventricular depolarization. A first backward-direction deviation in the QRS complex is referred to as the Q wave. A first forward-direction deviation in the QRS complex is referred to as the R wave. A backward-direction deviation after the R wave is referred to as the S wave. A waveform having a rounded and blunt top and appearing after the QRS complex is the T wave, and represents a state of ventricular repolarization. A complete waveform including each wave mentioned above is referred to as a beat. In a segment of a sufficiently-long signal, although an electrocardiography signal between two complete waveforms (beats) shows in a shape of waves, but a fluctuation degree of these waves is far less than that of waves in the QRS complex (including the Q wave, R wave and S wave). That is, the electrocardiography signal between the two complete waveforms (beats) is stationary. Therefore, in this application, an electrocardiography signal between every two complete waveforms (beats) in a segment of an electrocardiography signal, that is, a segment of a signal in FIG. 1 seeming to have a stationary fluctuation degree, may be referred to as a "stationary-segment signal".

In addition, it should be further noted that disturbance of electromyography noise, motion noise, etc., in the stationary-segment signal in the electrocardiography signal is most severe. Normally, in an electrocardiography signal having a desirable noise situation, an image of the stationary-segment signal should be gentle and should not have frequent and short wave peaks shown in FIG. 1. Since such noise disturbance is aliased with the electrocardiography signal, the electrocardiography signal distorts, and a waveform of the entire electrocardiography signal is obscure, which can affect identification of each electrocardiography wave band. Thus, a diagnostic result obtained by a medical worker based on the electrocardiography signal will also be effected. Therefore, selection of a proper method for removing noise has vital significance for electrocardiography.

In existing noise removal algorithms, some may have extremely little noise removal effects on the stationary-segment signal, and some may have some noise removal effects on the stationary-segment signal but may enable more disturbance in the QRS complex in the electrocardiography signal. Therefore, how to effectively remove noise from the stationary-segment signal in a case that disturbance in the QRS complex is minimized as much as possible is a problem emphatically to be resolved by this application.

(2) Electromyography (electromyography, EMG) noise

Electromyography noise, also referred to as an electromyography noise signal, is noise caused by a human activity and muscle tension, that is, superposition of muscle fibers in a motion unit action potential (MUAP) in a human body in time and space. A muscle activity in a head and neck is the main source of EEG disturbance, and a muscle activity below the neck generally does not cause much disturbance in the EEG. Such disturbance is small in amplitude but high in frequency. The frequency ranges from 5 Hz to 2000 Hz, and is shown as an irregular and rapidly-changing waveform.

(3) Motion noise

Motion noise is caused by a slight motion of a human body and has a main feature of abrupt change performance. A frequency generally ranges from 3 Hz to 14 Hz, and last for short time.

(4) Sampling point and sampling frequency

An electrocardiography signal is generally a bioelectricity signal of a body surface extracted by an electrocardiography device by using an electrode. The acquired electrocardiography signal is composed of a plurality of sampling points in time order. A value of the sampling point is an intensity or energy value of a bioelectricity signal of the body surface during acquisition. The plurality of sampling points are arranged in order of sampling time to form electrocardiography.

A sampling frequency refers to a number of points per second at which a recorder acquires a voltage of the electrocardiography signal. The higher the sampling frequency is, the less distortion of an electrocardiography waveform is, and the more accurate description by acquired data on continuous electrocardiography waveforms. When the sampling frequency is too low, amplitudes of the Q wave, the R wave and the S wave decrease, waveforms are stepped, and the electrocardiography will lose some significant information. Thus, it is necessary to apply a proper sampling frequency.

(5) Electrocardiography acquisition device

In an embodiment of this application, the electrocardiography acquisition device is a device for acquiring and analyzing an electrocardiography signal or another device related to acquisition and processing of the electrocardiography signal. The electrocardiography acquisition device may be an electrocardiography acquisition device, an electrocardiography machine, a wearable device or terminal having an electrocardiography sensor, or the like.

(6) High-pass filter and low-pass filter

A filter is a device that filters waves and is a circuit allowing a signal inside a frequency band to pass through but prevent a signal outside such a frequency band from passing through.

The filters are mainly low-pass filters and high-pass filters. The working principle is that an inductor prevents a high-frequency signal from passing through but allows a low-frequency signal to pass through, while a capacitor prevents the high-frequency signal from passing through but allows the low-frequency signal to pass through. The low-pass filter does not allow a to-be-cut-off high-frequency signal to pass through capacitive absorption and an inductive obstacle by using principles that a capacitor allows a high-frequency signal to pass through but prevents a low-frequency signal from passing through, and an inductor allows a low-frequency signal to pass through but prevents a high-frequency signal from passing through. A low-frequency signal required to pass through is allowed to passing through high resistance of the capacitor and low resistance of the inductor. Similarly, the high-pass filter prevents a low-frequency signal from passing through but allows a high-frequency signal to pass through.

(7) Quartiles and outliers

A quartile (Quartile) refers to each of numerical values at positions of three division points in a case that all numerical values are arranged in ascending order and divided into four equal parts in statistics. The quartiles are mostly applied for boxplot drawing in statistics. The quartiles are values at 25% and 75% positions after a set of data is sorted. Quartiles are data of which each part contains 25% data after all data is equally divided into 4 parts by 3 points. Obviously, a middle quartile is a median (generally represented by Q2), such that common quartiles are a numerical value at a 25% position (referred to as a lower quartile and generally represented by Q1) and a numerical value at a 75% position (referred to as an upper quartile and generally represented by Q3).

In a segment of a sequence of number, outliers in the sequence of number can be generally computed by virtue of quartiles. Generally, Q3+k(Q3-Q1) may be taken as a threshold, and values in the sequence of number that are greater than the threshold, that is, values in the sequence of number that are significantly greater than other numerical values are outliers. In a case that K is 1.5, the above threshold is the moderate outlier threshold, and numerical values in the sequence of number greater than the moderate outlier threshold are moderate outliers. In a case that K is 3, the above threshold is an extreme outlier threshold, and numerical values in the sequence of number greater than the extreme outlier threshold are extreme outliers.

For example, assuming that sequence of number [2, 3, 5, 6, 6, 7, 8, 9, 50, 75] including 10 numerical values is constructed, most (8) of numerical values fall within interval [0, 10], except for two numerical values, that is, 50 and 75. Through a method for computing a quartile, it can be computed that in the sequence of number, a lower quartile is $Q1=3\times0.25+5\times0.75=4.5$, a median is $Q2=(7+8)/2=7.5$, and an upper quartile is $Q3=9\times0.75+50\times0.25=19.25$. It can be seen in combination with the foregoing description that a moderate outlier threshold in the above sequence of number is $Q3+1.5(Q3-Q1)=33.375$. Thus, in the above sequence of number, numerical values greater than 33.375, that is, 50 and 75 in the sequence of number, are outliers.

Further, these numerical values are plotted in the form of dots in a two-dimensional rectangular coordinate system, and a broken line graph shown in FIG. 2 can be obtained. As shown in FIG. 2, except for line segments between points A, B, C and D, a fluctuating degree of a broken line graph formed by the other line segments is gentle (or an absolute value of slope between two adjacent points is less), but a fluctuating degree of a broken line graph formed by line segments AB, BC and CD is significantly greater (or an absolute value of slope between adjacent points of points A, B, C and D is less). It can be understood that an image of the electrocardiography signal has the same features as the image shown in FIG. 2. That is, the electrocardiography signal also has a stationary signal segment and a signal segment having an obvious waveform. In this application, when noise is removed from a stationary segment of an electrocardiography signal, whether a signal sampling point is a sampling point required to undergo noise removal is determined with reference to an amplitude of the sampling point and an amplitude of a sampling point closer to the sampling point and in combination with a quartile method such that noise can be removed from a stationary-segment signal in the electrocardiography signal without disturbing wave peaks of a P wave, a T wave and an R wave in the electrocardiography signal. Details can be obtained with reference to the following description and will not be described herein.

As a comprehensive representation of an electrical activity of a heart on a surface skin of a human body, the electrocardiography (electrocardiography, ECG)/electrocardiography signal contains a wealth of physiological and pathological information that reflects a heart rhythm and its electrical conduction, and thus can objectively reflect physiological conditions of various parts of the heart to some extent. The electrocardiography signal currently is one of the important methods for checking and diagnosing cardiovascular diseases in a non-invasive manner, and is an important basis for assessing whether the cardiac function is desirable.

In a process of acquiring an electrocardiography signal by professional in a hospital or an inexperienced home user, much noise appears in the acquired electrocardiography signal due to disturbance from various factors such as turn-over, motion and a power line. Especially electrocardiography of a user who is not stationary acquired by a wearable electrocardiography device may include plenty of noise. Such noise undoubtedly will cause signal quality problems, and will disturb identification of features affecting the electrocardiography, thereby reducing accuracy and reliability of electrocardiography diagnosis. Therefore, it is highly necessary to remove noise from the electrocardiography signal. Noise such as electromyography noise and motion noise in the electrocardiography signal is found in a full frequency band of the electrocardiography signal. Effects of such noise are small but difficult to eradicate. Effects of such noise in a stationary segment of the signal are more apparent. When such noise in the electrocardiography signal is removed through a conventional method for removing noise, wave peaks of a P wave, a T wave and an R wave in the electrocardiography signal are likely to be disturbed. If the P wave, T wave and R wave serving as critical features in the electrocardiography signal are effected, the electrocardiography signal will lose a reference value.

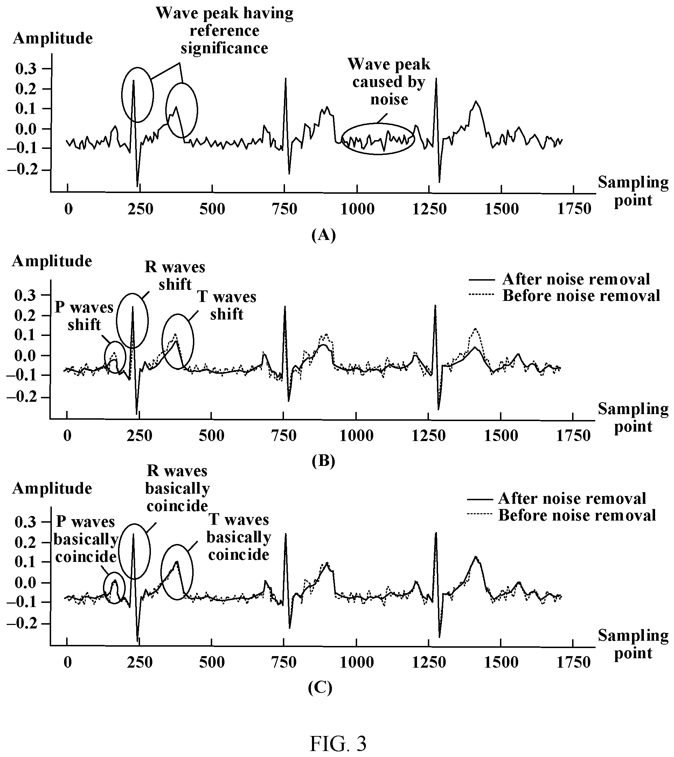

FIG. 3 is a schematic diagram of a noise removal result of an electrocardiography signal according to an embodiment of this application. FIG. 3(A) shows an image of an original electrocardiography signal that does not undergo noise removal. FIG. 3(B) shows an image of an electrocardiography signal obtained after noise is removed from the original electrocardiography signal through an existing method for removing noise. FIG. 3(C) shows an image of an electrocardiography signal obtained after noise is removed from the original electrocardiography signal through a method for removing noise provided in this application.

As shown in FIG. 3(A), the original electrocardiography signal may be an electrocardiography signal acquired by a wearable device having an electrocardiography sensor, such as a smart band and a smart watch. The wearable device may also be a dynamic electrocardiography instrument widely used by institutions such as hospitals, that is, a conventional twelve-lead electrocardiography signal acquisition instrument. It should be understood that the original electrocardiography signal is an electrocardiography signal that is directly acquired by the device, does not undergo noise removal, but may be subject to pre-processing. It can be seen in combination with the foregoing description that the electrocardiography signal is a weak signal having strong non-linearity, non-stationarity and randomness, and is extremely likely to be effected by in-vivo and in-vitro environments when being acquired, such as motions of human limbs, respiration and electromagnetic disturbance in the surroundings. Thus, the electrocardiography signal directly acquired is accompanied by plenty of noise. Such noise may appear as frequent and short wave peaks in the image of the electrocardiography signal, that is, wave peaks caused by noise shown in FIG. 3(A). It can be seen from FIG. 3(A) that the effect of such noise is more obvious in the stationary-segment signal of the electrocardiography signal. In addition to these wave peaks, some wave peaks that are not caused by noise, are persistent, and has reference significance may exist in the electrocardiography signal, such as the P wave and R wave mentioned before. Therefore, in an ideal situation, when noise is removed from the above original electrocardiography signal, frequent and short wave peaks, that is, wave peaks caused by noise, should be suppressed without disturbing the wave peaks having reference significance. In this way, noise, especially noise in the stationary-segment signal, can be effectively removed from the electrocardiography signal without affecting the reference value of the electrocardiography signal.

FIG. 3(B) shows an image of an electrocardiography signal obtained after noise is removed from the original electrocardiography signal through an existing method for removing noise. It should be noted that for convenience of comparison with the original electrocardiography signal, in FIG. 3(B), a solid line represents the image of the electrocardiography signal obtained after noise removal, and a dashed line represents an image of an electrocardiography signal (that is, the electrocardiography signal shown in FIG. 3(A)) before noise removal. It can be seen that a noise situation of an electrocardiography signal obtained by removing noise from an original electrocardiography signal through an existing method for removing noise is significantly improved, frequent and short wave peaks in an image of the electrocardiography signal caused by noise are significantly reduced, and a change tendency of a stationary-segment signal in the electrocardiography signal is simpler and clearer.

However, in the image shown in FIG. 3(B), if images of the electrocardiography signal before and after noise removal are compared, it is not difficult to see that although the existing method for removing noise can have some noise removal effects on the original electrocardiography signal, in the new electrocardiography signal obtained by removing noise from the original electrocardiography signal, wave peaks having vital reference significance of the original electrocardiography signal shift (distort). As shown in FIG. 3(B), in a first beat of the electrocardiography signal, a peak value of an R wave, a peak value of a P wave and a peak value of a T wave of the electrocardiography signal after noise removal all less than a peak value of the R wave, the peak value of the P wave and the peak value of the T wave of the electrocardiography signal before noise removal. Medically, waves having reference significance, such as the P wave and the T wave can generally reflect a health condition of a heart of a person. When these waves distort, a diagnostic result obtained by a medical worker based on an electrocardiography signal on which these waves are located may be inaccurate, and may even compromise life safety of a patient in a severe case.

Therefore, to resolve the above problems, this application provides a method for removing noise from a signal, and an electronic device. According to the method, by virtue of a continuous abrupt change performance of a wave peak having reference significance in an electrocardiography signal, whether the signal point is a point on a signal wave having reference significance can be identified during noise removal, and whether to remove noise from the signal point is determined. The method is implemented, so that noise can be removed from an electrocardiography signal without affecting a wave having reference significance in the electrocardiography signal. That is, noise can be effectively removed from the electrocardiography signal on the premise that a reference value of the electrocardiography signal is guaranteed.

FIG. 3(C) shows the image of the electrocardiography signal obtained after noise is removed from the original electrocardiography signal through a method for removing noise provided in this application. Similarly, for convenience of comparison with the original electrocardiography signal, in FIG. 3(C), a solid line represents the image of the electrocardiography signal obtained after noise removal, and a dashed line represents an image of an electrocardiography signal (that is, the electrocardiography signal shown in FIG. 3(A)) before noise removal. It can be seen that a noise situation of an electrocardiography signal obtained by removing noise from an original electrocardiography signal through a method provided in this application is also significantly improved, frequent and short wave peaks in an image of the electrocardiography signal caused by noise are significantly reduced, and a change tendency of a stationary-segment signal in the electrocardiography signal is simpler and clearer. In addition, the method for removing noise provided in this application further guarantees the reference value of the electrocardiography signal based on the noise removal effect on the stationary-segment signal is guaranteed, that is, after noise is removed from the electrocardiography signal, coincidence of key wave peaks of a signal after noise removal and an original signal before noise removal is extremely high. In a first beat of the electrocardiography signal shown in FIG. 3(C), an R wave, a P wave and a T wave of the electrocardiography signal after noise removal substantially coincide with an R wave, a P wave, a T wave of the electrocardiography signal before noise removal.

An electronic device provided in an embodiment of this application will be first introduced below.

This electronic device may be a mobile phone, a tablet computer, a wearable device such as a smart bracelet and a smart watch, an in-vehicle device, an augmented reality (augmented reality, AR)/virtual reality (virtual reality, VR) device, a notebook computer, an ultra-mobile personal computer (ultra-mobile personal computer, UMPC), a netbook, a personal digital assistant (personal digital assistant, PDA), a dedicated camera (such as a digital single lens reflex or a cube camera), etc. A specific type of the electronic device is not limited in this application.

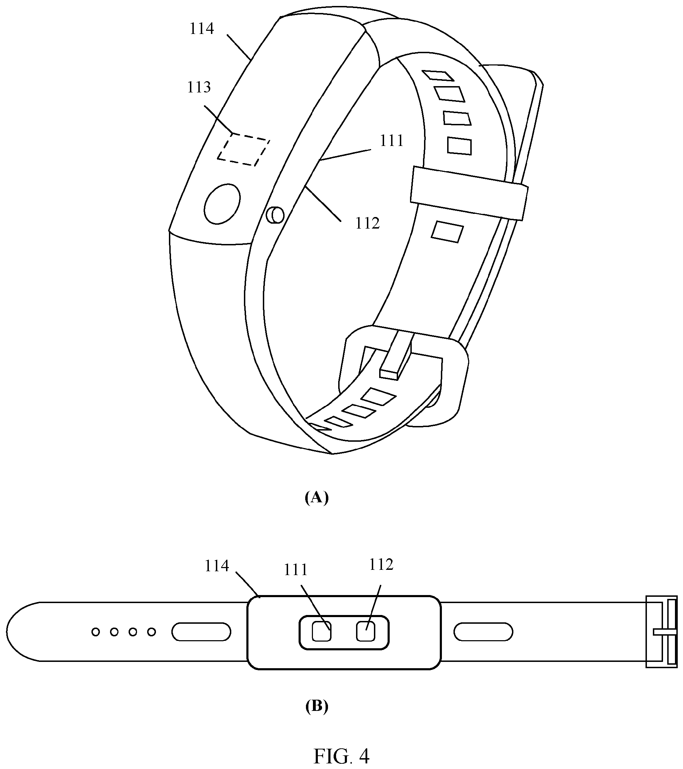

In a case that the above electronic device is a wearable device such as a smart bracelet and a smart watch, an appearance of the electronic device can be obtained with reference to FIG. 4. FIG. 4(A) shows a specific pattern of the electronic device worn on a wrist of a user. FIG. 4(B) shows a specific pattern of a back (that is, a side tightly attached to a skin of a user when worn) of the electronic device.

As shown in FIG. 4, the above electronic device may be further provided with an electrocardiography sensor to acquire electrocardiography data of the user. The electrocardiography sensor generally includes two electrodes configured to acquire electrocardiography signals. In FIG. 4, two electrodes (that is, an electrode 111 and an electrode 112) of the electrocardiography sensor are both arranged on a back of the electronic device. The electronic device may internally include a data conversion module 113. The data conversion module 113 may carry out analog-to-digital conversion on analog electrocardiography signals acquired by using the electrodes 111 and 112, such that discrete digitized electrocardiography signals are obtained. Then, a processing module (not shown in FIG. 4) in the electronic device may remove, through the method for removing noise from a signal in an embodiment of this application, noise from the digitized electrocardiography signal serving as an electrocardiography signal to undergo noise removal, such that an electrocardiography signal after noise removal is obtained.

It should be understood that a user may press a finger against a dial 114 of the electronic device to enable the electrodes 112 and 111 to make contact with an arm of the user when acquiring an electrocardiography signal by the above electronic device.

Optionally, the above electronic device may further analyze the electrocardiography signal after noise removal, and an analysis result is obtained. Further, the above electronic device may further output an analysis result by using an output apparatus, such as a display and a loudspeaker.

The above electronic device may further transmit the electrocardiography signal to undergo noise removal to a terminal or a server to which the electronic device is bound. Noise is removed from the electrocardiography signal to undergo noise removal by the terminal or the server through the electrocardiography method for removing noise in an embodiment of this application, and the electrocardiography signal after noise removal is obtained. The terminal or the server may transmit the electrocardiography signal after noise removal or an analysis result obtained by analyzing the electrocardiography signal after noise removal to the above electronic device.

The above electronic device configured with an electrocardiography sensor can monitor electrocardiography signal data of a wearer in real-time or periodically to monitor a physical condition of the wearer. It can be understood that the electronic device in an embodiment of this application is a smart terminal device. This electronic device has one or more functions of reminding, navigation, calibration, monitoring, interaction, etc., in addition to time indication. A display form of the smart watch includes a pointer, a number, an image, etc.

It should be noted that a schematic diagram of an appearance of an electronic device shown in FIG. 4 does not constitute a specific limitation on the terminal device. In some other embodiments of this application, the appearance of the electronic device 100 may differ from that shown in FIG. 4. For example, in some embodiments, the dial 114 of the above electronic device may be circular. For another example, one electrode 111 of the electrocardiography sensor of the above electronic device may be arranged on the back of the electronic device, and the other electrode 112 may be arranged on the side of the electronic device. A user may press a finger against the electrode 112 when acquiring the electrocardiography signal, and the electrode 111 makes contact with an arm of the user.

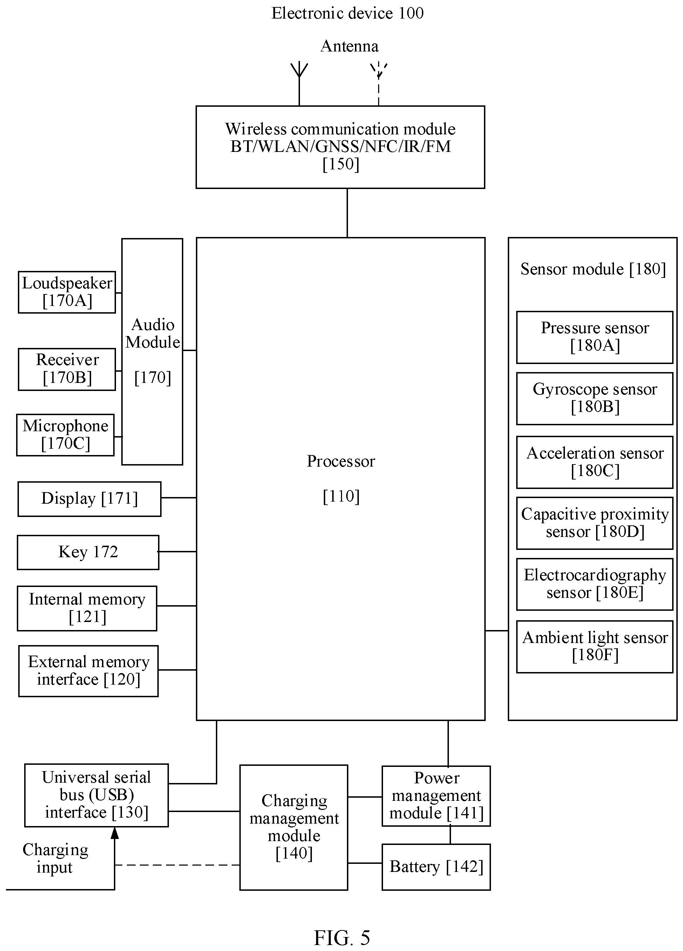

For example, FIG. 5 shows a structure of the above electronic device.

As shown in FIG. 5, the electronic device 100 can perform a method for removing noise from a signal provided in an embodiment of this application. Specifically, as shown in FIG. 5, the electronic device 100 may include a processor 110, an external memory interface 120, an internal memory 121, a universal serial bus (universal serial bus, USB) interface 130, a charging management module 140, a power management module 141, a battery 142, an antenna, a wireless communication module 150, an audio module 170, a loudspeaker 170A, a telephone receiver 170B, a microphone 170C, a camera 172, a display 171, a sensor module 180, etc. The sensor module 180 may include a pressure sensor 180A, a gyroscope sensor 180B, an acceleration sensor 180C, a capacitive proximity sensor 180D, an electrocardiography sensor 180E, an ambient light sensor 180F, etc.

It can be understood that a schematic structure in an embodiment of this application does not constitute a specific limitation on the electronic device 100. In some other embodiments of this application, the electronic device 100 may include more or fewer components than those shown in the figures, combine some components, split some components, or differently arrange components. The components shown in the figures may be implemented as hardware, software, or their combination.

The processor 110 may include one or more processing units. For example, the processor 110 may include an application processor (application processor, AP), a modem, a graphics processing unit (graphics processing unit, GPU), an image signal processor (image signal processor, ISP), a controller, a video codec, a digital signal processor (digital signal processor, DSP), a baseband processor, and/or a neural-network processing unit (neural-network processing unit, NPU). Different processing units may be separate devices, or may be integrated into one or more processors. The controller may generate an operation control signal according to instruction operation code and a time-sequence signal, and control instructions to be obtained and performed.

A memory may be further arranged in the processor 110 and is configured to store instructions and data. In some embodiments, the memory in the processor 110 is a buffer memory. This memory may store instructions or data recently used or cyclically used by the processor 110. If the processor 110 is required to use these instructions or data again, the processor may directly invoke the instructions or the data from the memory. Repeated access is avoided, and waiting time of the processor 110 is reduced, thereby improving system efficiency. Moreover, the processor 110 may further store data received by the electronic device 100 from other electronic devices.

For example, in some embodiments of this application, the processor 110 may control the electronic device 100 to transmit the electrocardiography signal to undergo noise removal to a terminal or a server to which the processor is bound. Noise is removed from the electrocardiography signal to undergo noise removal by the terminal or the server through the electrocardiography method for removing noise in an embodiment of this application, and the electrocardiography signal after noise removal is obtained. The electronic device 100 may receive the electrocardiography signal after noise removal transmitted by the terminal or server, or an analysis result obtained by analyzing the electrocardiography signal after noise removal by the terminal or server.

In a case that the electronic device 100 is a smart watch, the processor 110 controls a dial and a base of the smart watch to obtain initial electrocardiography signals. Optionally, the processor 110 includes a filtering device or circuit. The filtering device or circuit filters the above initial electrocardiography signals to filter out high-and-low-frequency-band signals from the initial electrocardiography signals and retain signals to be analyzed. Further, the processor 110 further removes, through the method for removing noise from a signal in an embodiment of this application, noise from the above signals obtained through filtration, and an electrocardiography signal after noise removal is obtained.

In some embodiments, the processor 110 may include one or more interfaces. The interface may include an inter-integrated circuit (inter-integrated circuit, I2C) interface, an inter-integrated circuit sound (inter-integrated circuit sound, I2S) interface, a pulse code modulation (pulse code modulation, PCM) interface, a universal asynchronous receiver/-transmitter (universal asynchronous receiver/transmitter, UART) interface, a mobile industry processor interface (mobile industry processor interface, MIPI), a general-purpose input/output (general-purpose input/output, GPIO) interface, a subscriber identity module (subscriber identity module, SIM) interface, a universal serial bus (universal serial bus, USB) interface, a micro USB interface, a USB Type C interface, etc.

The external memory interface 120 may be configured to be connected to an external storage card such as a micro SD card to expand a storage capability of the electronic device 100. The external storage card is in communication with the processor 110 through the external memory interface 120 to implement a data storage function, for example, store a file such as audio or video in the external storage card.

The internal memory 121 may be configured to store computer-executable program code, and the executable program code includes instructions. The internal memory 121 may include a program storage region and a data storage region. The program storage region may store an operating system, an application required by at least one function (such as a sound play function or an image playing function), etc. The data storage region may store data (such as audio data and an address book), etc., created when the electronic device 100 is used. In addition, the internal memory 121 may include a high-speed random access memory, and may further include a nonvolatile memory, such as at least one magnetic disk storage device, a flash storage device, or a universal flash storage (universal flash storage, UFS). The processor 110 runs the instructions stored in the internal memory 121 and/or the instructions stored in the memory arranged in the processor to perform various function applications and data processing of the electronic device 100.

The USB interface 130 may be configured to be connected to a charger to charge the electronic device 100, or may be used for data transmission between the electronic device 100 and a peripheral device. The interface may be further configured to be connected to a headset to play audio through the headset. This interface may be further configured to be connected to another electronic device such as an AR device.

It can be understood that a schematic interface connection relationship between all modules in an embodiment of this application is merely schematic description, and does not constitute a limitation on the structure of the electronic device 100. In some other embodiments of this application, the electronic device 100 may use an interface connection manner different from that in the above embodiments, or use combinations of a plurality of interface connection manners.

A wireless communication function of the electronic device 100 may be implemented by using an antenna, a wireless communication module 150, a modem, a baseband processor, etc. The electronic device 100 may be in wireless communication with other electronic devices by using the wireless communication module 150. The antenna is configured to transmit and receive electromagnetic wave signals. Each antenna of the electronic device 100 may be configured to cover one or more communication frequency bands. Different antennas may be further multiplexed to improve utilization of the antennas. For example, an antenna may be multiplexed as a diversity antenna of a wireless local area network. In some other embodiments, the antennas may be used in combination with a tuning switch.

The wireless communication module 150 may be one or more devices that integrate at least one communication processing module. The wireless communications module 150 may provide a solution to wireless communication applied to the electronic device 100, including a wireless local area network (wireless local area networks, WLAN) (such as a wireless fidelity (wireless fidelity, Wi-Fi) network), Bluetooth (bluetooth, BT), a global navigation satellite system (global navigation satellite system, GNSS), frequency modulation (frequency modulation, FM), near field communication (near field communication, NFC), and an infrared (infrared, IR) technology. The wireless communication module 150 may be one or more devices that integrate at least one communication processing module. The wireless communication module 150 receives an electromagnetic wave through an antenna, modulates a frequency of the electromagnetic wave signal and filters the electromagnetic wave signal, and sends the processed signal to the processor 110. The wireless communication module 150 may further receive a to-be-transmitted signal from the processor 110, modulate a frequency of the to-be-transmitted signal and amplify the to-be-transmitted signal, and convert the to-be-transmitted signal to an electromagnetic wave by the antenna for radiation.

In some embodiments, the electronic device 100 may be in communication with other electronic devices by using the wireless communication module 150.

The electronic device 100 may implement an audio function by using the audio module 170, the loudspeaker 170A, the telephone receiver 170B, the microphone 170C, the application processor, etc., such as music play and sound recording.

The audio module 170 is configured to convert digital audio information into analog audio signal output, and is also configured to convert analog audio input into a digital audio signal. The audio module 170 may be further configured to encode and decode audio signals. In some embodiments, the audio module 170 may be arranged in the processor 110, or some function modules of the audio module 170 are arranged in the processor 110.

The loudspeaker 170A, also referred to as a "speaker", is configured to convert an electrical audio signal into a sound signal. The electronic device 100 may listen to the music or answer a hands-free call by using the loudspeaker 170A.

The telephone receiver 170B, also referred to as a "receiver", is configured to convert an electrical audio signal into a sound signal. When the electronic device 100 answers a call or receives voice information, the telephone receiver 170B may be put close to a human ear to receive a voice.

The microphone 170C, also referred to as a "voice tube" or "mike", is configured to convert a sound signal into an electrical signal. When making a call or sending voice information, a user may make a sound approaching the microphone 170C through the mouth of the user to input a sound signal into the microphone 170C. The electronic device 100 may be provided with at least one microphone 170C. In some other embodiments, the electronic device 100 may be provided with two microphones 170C to acquire a sound signal and implement a noise removal function. In some other embodiments, the electronic device 100 may be provided with three, four, or more microphones 170C to acquire a sound signal, reduce noise, recognize a sound source, implement a directional recording function, etc.

The key 172 includes a power key, a volume key, etc. The key 172 may be a mechanical key, or a touch key. The electronic device 100 may receive key input, and generate key signal input related to user settings and function control of the electronic device 100.

The electronic device 100 implements a display function by using the GPU, the display 171, the application processor, etc. The GPU is a microprocessor for image processing and connects the display 171 and the application processor. The GPU is configured to perform mathematical and geometric computation, and is configured to render graphics. The processor 110 may include one or more GPUs that perform program instructions to generate or change display information.

The display 171 is configured to display an image, a video, etc. The display 71 includes a display panel. A liquid crystal display (liquid crystal display, LCD), an organic light-emitting diode (organic light-emitting diode, OLED), an active-matrix organic light emitting diode (active-matrix organic light emitting diode, AMOLED), a flexible light-emitting diode (flex light-emitting diode, FLED), a Miniled, a MicroLed, a Micro-oLed, a quantum dot light emitting diodes (quantum dot light emitting diodes, QLED), etc., may be used as the display panel. In some embodiments, the electronic device 100 may include 1 or N displays 171, and N is a positive integer greater than 1.

The pressure sensor 180A is configured to sense a pressure signal, and can convert the pressure signal into an electrical signal. In some embodiments, the pressure sensor 180A may be arranged on the display 71. There are many types of pressure sensors 180A, such as a resistive pressure sensor, an inductive pressure sensor, and a capacitive pressure sensor. The capacitive pressure sensor may be a parallel plate including at least two electrically-conductive materials. In a case that a force is applied to the pressure sensor 180A, capacitance between electrodes changes. The electronic device 100 determines pressure strength based on a change in the capacitance. When a touch operation acts on the display 71, the electronic device 100 detects strength of the touch operation according to the pressure sensor 180A. The electronic device 100 may further compute a touch position based on a detection signal of the pressure sensor 180A. In some embodiments, touch operations that are applied to the same touch position but have different touch operation strength may correspond to different operation instructions. For example, when a touch operation of which touch operation strength is less than a first pressure threshold acts on a short message application icon, an instruction of viewing a short message is performed. When a touch operation of which touch operation strength is greater than or equal to the first pressure threshold acts on the short message application icon, an instruction of creating a new short message is performed.

The gyroscope sensor 180B may be configured to determine a motion posture of the electronic device 100. In some embodiments, an angular velocity of the electronic device 100 around three axes (that is, x, y and z axes) may be determined by a gyroscope sensor 180B. The gyroscope sensor 180B may be configured to prevent a shake during photographing. For example, when a shutter is pressed, the gyroscope sensor 180B measures a shake angle of the electronic device 100, and computes, based on the angle, a distance by which a lens module is required to compensate. Moreover, the lens cancels the shake of the electronic device 100 through a reverse motion, such that the shake is prevented. The gyroscope sensor 180B may be further configured for navigation and motion sensing game scenarios.

The acceleration sensor 180C may measure an acceleration value of the electronic device 100 in all directions (generally three axes). When the electronic device 100 is stationary, an amplitude and a direction of gravity can be measured. The acceleration sensor may be further configured to recognize a posture of the electronic device, and is applied to applications such as switch between horizontal and vertical screens and a pedometer.

The capacitive proximity sensor 180D may include, for example, a light-emitting diode (LED) and an optical detector such as a photodiode. The light-emitting diode may be an infrared light-emitting diode. The electronic device 100 emits infrared light by using the light-emitting diode. The electronic device 100 detects infrared reflected light from a nearby object by using the photodiode. When sufficient reflected light is detected, it can be determined that an object exists near the electronic device 100. When insufficient reflected light is detected, the electronic device 100 may determine that no object exists near the electronic device 100. The electronic device 100 may detect, by using the capacitive proximity sensor 180D, that a user holds the electronic device 100 close to an ear for a call, such that automatic screen-off is implemented to save power. The capacitive proximity sensor 180D may be further used in a leather case mode or a pocket mode to automatically unlock or lock the screen.

The electrocardiography sensor 180E generally includes two electrodes configured to acquire electrocardiography signals. A sinus node in a human heart rhythmically controls systole and diastole to pump blood to a torso. This control signal is an electrical signal (human nerve signals are shown as electrical signals on nerves), can gradually diffuse to the body surface and can be measured at the skin by using electrodes. The electrocardiography sensor 180E may detect bioelectricity in a case of making contact with human skin, and convert the bioelectricity into electrical signals. These electrical signals may be digitally processed by the processor 110 to be converted into digitized signals. The processor may further process these digitized signals in combination with the GPU, obtain accurate and detailed electrocardiography, and display the electrocardiography on the display 171.

The ambient light sensor 180F is configured to perceive ambient light brightness. The electronic device 100 may adaptively adjust luminance of the display 171 according to the perceived ambient light brightness. The ambient light sensor 180F may be further configured to automatically adjust white balance during photographing. The ambient light sensor 180L may further cooperate with the capacitive proximity sensor 180D to detect whether the electronic device 100 is in a pocket, so as to prevent an accidental touch.

The charging management module 140 is configured to receive charging input from the charger. The charger may be a wireless charger or a wired charger. In some embodiments of wired charging, the charging management module 140 may receive charging input of the wired charger through the USB interface 130. In some embodiments of wireless charging, the charging management module 140 may receive wireless charging input by using a wireless charging coil of the electronic device 100. When the charging management module 140 charges a battery 142, the power management module 141 may also supply power to the electronic device.

The power management module 141 is configured to be connected to the battery 142, the charging management module 140 and the processor 110. The power management module 141 receives input of the battery 142 and/or the charging management module 140 to supply power to the processor 110, the internal memory 121, the display 171, the wireless communications module 150, etc. The power management module 141 may be further configured to monitor parameters such as battery capacity, a battery cycle count, a battery state of health (electric leakage and impedance). In some other embodiments, the power management module 141 may be arranged in the processor 110. In some other embodiments, the power management module 141 and the charging management module 140 may be also arranged in a same device.

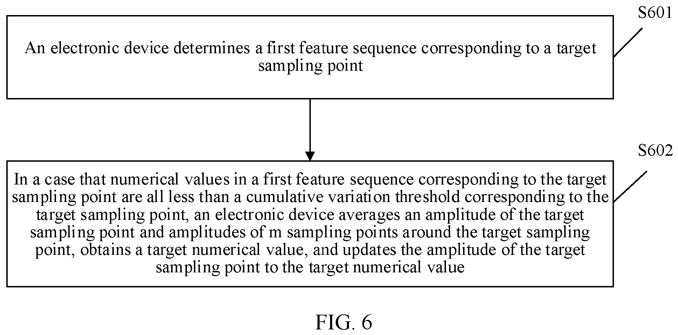

FIG. 6 is a flow diagram of a method for removing noise from a signal according to an embodiment of this application. According to the method, by virtue of a continuous abrupt change performance of a wave peak having reference significance in an electrocardiography signal, whether the signal point is a point on a signal wave having reference significance can be identified during noise removal, and whether to remove noise from the signal point is determined. The method is implemented, so that noise can be removed from an electrocardiography signal without affecting a wave having reference significance in the electrocardiography signal. That is, noise can be effectively removed from the electrocardiography signal on the premise that a reference value of the electrocardiography signal is guaranteed. As shown in FIG. 6, the method provided in an embodiment of this application may include but is not limited to the following steps:

S601: An electronic device determines a first feature sequence corresponding to a target sampling point.

The above electronic device may be the electronic device shown in FIG. 4 or the electronic device 100 in the foregoing description.

The above target sampling point is a sampling point in a signal to undergo noise removal. This signal to undergo noise removal may be a signal directly acquired by the above electronic device, or a signal acquired by another device and transmitted to the above electronic device.

Specifically, the above signal to undergo noise removal may be an electrocardiography signal. This electrocardiography signal may be a bioelectricity signal of a biological body surface extracted by the above electronic device by using an electrode. The acquired electrocardiography signal is composed of a plurality of sampling points ordered according to sampling time. In the electrocardiography signal, a value of the sampling point is an intensity or energy value of a bioelectricity signal of the body surface during acquisition. The plurality of sampling points are arranged in order of sampling time to form electrocardiography having a fluctuation degree.

In an optional implementation, before performing S601, the electronic device further high-pass-filters and low-pass-filters an initial signal, and obtains the above signal to undergo noise removal. This initial signal is an electrical signal that represents a heart rhythm of a user and is acquired by an electronic device. A high-pass filter and a low-pass filter used by the electronic device may be multi-order filters. These filters can filter out higher and lower frequency bands, especially those frequency bands that may include artifacts, of an initial signal acquired by the electronic device. Subsequent analysis is facilitated, a frequency range of a signal to be analyzed is retained, and processing efficiency of a subsequent processing process is improved. Specifically, in a high-frequency band, the electronic device may use a low-pass filter having a cut-off frequency lower than an alternating-current electrical frequency (50 Hz or 60 Hz) to avoid power frequency disturbance. In a low-frequency band, that is, a frequency band lower than 1 Hz, the electronic device may use a high-pass filter that retains a highest frequency band of a signal of interest.

It should be understood that normal electrocardiography signals are generally shown as wave peaks (or wave troughs) having different fluctuation degrees. In combination with the foregoing description, a few of wave peaks of which fluctuation degrees are particularly obvious, that is, the Q wave, the R wave and the S wave in the QRS complex, exist in the electrocardiography signal. The highest peak value R wave in the electrocardiography exists in the QRS complex. These electrocardiography signals are important reference features in electrocardiography signals. For example, when the Q wave is abnormal, it indicates that a patient may have a risk of a stale myocardial infarction. Therefore, preservation of authenticity of the QRS complex in the electrocardiography signals when noise is removed from the signal has vital significance for determination of an overall rhythm and frequency.

A QRS wave in the electrocardiography signal is generally the largest, most obvious and sharpest wave. This feature is mathematically referred to as singularity or abrupt change performance of the QRS wave and shown as an abrupt change in slope. That is, an amplitude difference between two adjacent sampling points on a QRS complex in the electrocardiography signal is extremely great compared with that of a stationary-segment signal in the electrocardiography signal. Thus, an absolute value of slope between two adjacent sampling points in the QRS complex is extremely great.

In addition, one wave can be regarded as two parts according to a change tendency, that is, a rising part (in which the amplitude of the sampling point is increasingly great) and a falling part (in which the amplitude of the sampling point is increasingly great). The wave peak rises and then falls. The wave trough falls and then rises. The amplitude of the sampling point in the QRS complex may change in a fixed tendency for longer time than that of the stationary-segment signal. For example, in a QS segment of the QRS complex, the amplitude of the signal persistently rises and then persistently falls. That is, in the electrocardiography signal, in a case that a segment of sub-signal is selected from the electrocardiography signal in a time window (for example, a time window including 30 sampling points) having a fixed length, if the segment of sub-signal is a signal completely located in the QRS complex, an amplitude of the segment of sub-signal is extremely likely to sharply rise or sharply fall. If the segment of sub-signal is a signal located in a stationary-segment signal, a change tendency of the amplitude of the segment of signal is extremely likely to repeatedly change. For example, the amplitude rises, falls, rises, falls..., and repeatedly changes in this way. Moreover, a change range (which is embodied in the image as whether a wave is sharp enough) of the segment of sub-signal may be extremely small.

Thus, the electronic device can carry out reference with one change tendency as a dimension, and can determine whether a signal is a signal on the QRS complex by viewing persistence and an abrupt change performance of a segment of signal determined by using a sampling point in a change tendency, that is, whether a segment of continuous sampling points exists in the segment of signal, whether a number of the sampling points is sufficient and amplitudes of the sampling points keep sharply rising or falling, and further determine whether the sampling point is a point on or around the QRS complex. Based on this, in the method, the above electronic device can construct, based on change a tendency and range of an amplitude of each sampling point and sampling points around the sampling point, a feature sequence reflecting whether the point is a sampling point on the QRS complex. The feature sequence may be referred to as a first feature sequence corresponding to the point.

With the above target sampling point as an example for description, the above first feature sequence corresponding to the target sampling point includes j elements. These j elements correspond to, in a one-to-one manner, j sampling points in the above signal to undergo noise removal. An element corresponding to any sampling point of the above j sampling points represent a sum of rising or falling ranges of all sampling points between the above any sampling point and a first sampling point. A falling or rising range of the any sampling point of the above j sampling points is equal to a difference between an amplitude of the above any sampling point and an amplitude of a sampling point after the above any sampling point. The above first sampling point is a sampling point that is located before the above any sampling point, has an opposite change tendency relative to the above any sampling point, and is closest to the above any sampling point.

The above expression "has an opposite change tendency" means that an amplitude of a sampling point before the first sampling point is greater than an amplitude of the first sampling point, but an amplitude of a sampling point after the first sampling point is less than the amplitude of the first sampling point; or means that the amplitude of the sampling point before the first sampling point is less than the amplitude of the first sampling point but the amplitude of the sampling point after the first sampling point is greater than the amplitude of the first sampling point. Specifically, on an image, the first sampling point may be a sampling point located on a wave peak or a wave trough in the above signal to be detected.

In addition, the above expression "sum of rising or falling ranges" means either a sum of rising ranges or a sum of falling ranges. That is, as observed from only one change tendency, whether a sufficient number of continuous sampling points of which amplitudes keep sharply rising exist in the above j sampling points or whether a sufficient number of continuous sampling points of which amplitudes keep sharply falling exist in the above j sampling points is determined.

Further, r mentioned above may be a positive integer greater than 1. In an optional implementation, j mentioned above may be determined according to a sampling frequency of the above signal to undergo noise removal. Specifically, j mentioned above may be equal to thirty percent of a sampling frequency of the above signal to undergo noise removal. For example, if the sampling frequency of the above signal with noise removal is 500, j mentioned above may be $500 \times 0.3 = 150$.

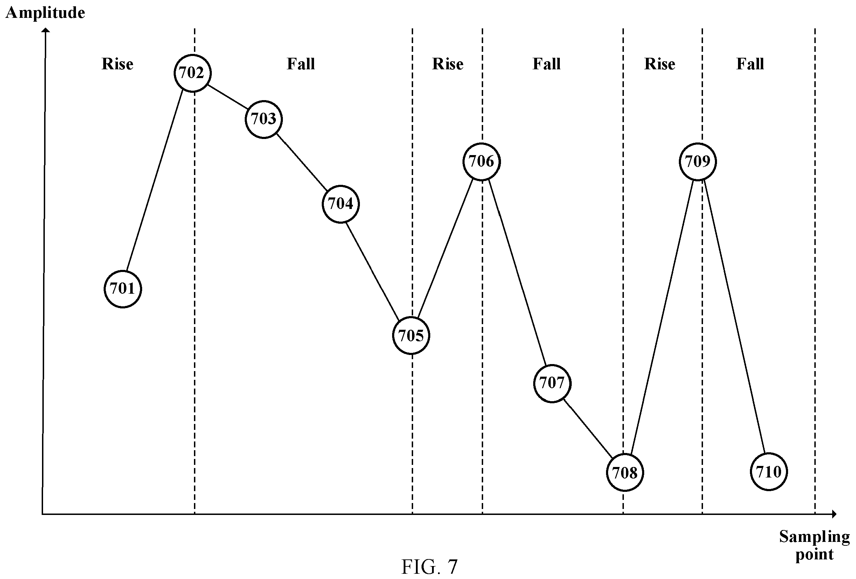

Derails can be obtained with reference to the broken line graph shown in FIG. 7. It is assumed herein that a curve in FIG. 7 is an image of a segment of sub-signal of the above signal to undergo noise removal. This sub-signal is composed of ten sampling points from sampling point 701 to sampling point 710. In a coordinate system shown in FIG. 7, a vertical axis represents an amplitude of the sampling point, and the amplitude of the sampling point located more upward is larger. Assuming that the sampling point 701 is the above target sampling point, it can be seen from FIG. 7 that the segment of the electrocardiography signal from the sampling point 701 to the sampling point 710 can be divided into 6 segments according to an amplitude change tendency, that is, a rising segment corresponding to the sampling point 701 to the sampling point 702, a falling segment corresponding to the sampling point 702 to the sampling point 705, a rising segment corresponding to the sampling point 705 to the sampling point 706, a falling segment corresponding to the sampling point 706 to the sampling point 708, a rising segment corresponding to the sampling point 708 to the sampling point 709, and a falling segment corresponding to the sampling point 709 to the sampling point 710. The change tendency of each sampling point depends on whether the sampling point is in a rising or falling segment. For example, if the sampling point 701 is in a rising segment, the change tendency of the sampling point 701 is rising. If the sampling point 704 is in a falling segment, the change tendency of the sampling point 704 is falling. The sampling points 702, 705, 706, 708 and 709 are critical points where the change tendency changes. Whether the change tendency rises or falls depends on the change tendency of the signal segment where the sampling point is located.

The sub-signal shown in FIG. 7 is analyzed herein by taking a falling tendency as a reference dimension. If the falling tendency is taken as the reference dimension, a change degree and speed of an image in a rising segment is not required to be considered. Thus, in a case of the sampling points in FIG. 7, a change tendency between all the sampling points in the rising segment corresponding to the sampling point 701 to the sampling point 702, the rising segment corresponding to the sampling point 705 to the sampling point 706, and the rising segment corresponding to the sampling point 708 to the sampling point 709 may be 0 by default. That is, if $x_{701} < x_{702}$ (where $x_t$ represents an amplitude corresponding to sampling point t, for example, $x_{701}$ represents an amplitude of the sampling point 701, and the same below), a change range from $x_{701}$ to $x_{702}$ is regarded as 0. Similarly, a change range from $x_{705}$ to $x_{706}$, and a change range from $x_{708}$ to $x_{709}$ are all 0.

In the falling segment corresponding to the sampling point 702 to the sampling point 705, the change tendency of the sampling point 703 to the sampling point 705 is a falling tendency. Thus, the sampling point 702 is a closest sampling point located before any of the sampling point 703 to the sampling point 705 and has an opposite change tendency to same. That is, the sampling point 702 is a "first sampling point" of the sampling point 703 to the sampling point 705. Sums of the falling ranges of the sampling point 703 to the sampling point 705 and the sampling point 702 is $x_{702}$-$x_{703}$, $x_{702}$-$x_{704}$ and $x_{702}$-$x_{705}$ respectively. Similarly, in the falling segment corresponding to the sampling point 706 to the sampling point 708, sums of the falling ranges of the sampling point 703 to the sampling point 705 and the sampling point 702 is $x_{06}$-$x_{707}$ and $x_{706}$-$x_{708}$ respectively. In the falling segment corresponding to the sampling point 709 to the sampling point 710, a sum of the falling ranges of the sampling point 710 and the sampling point 709 is $x_{709}$-$x_{710}$.

Then, j (where j is equal to 9) elements included in the first feature sequence corresponding to the sampling point 701 as the target sampling point are [0, $x_{702}$-$x_{703}$, $x_{702}$-$x_{704}$, $x_{702}$-$x_{705}$, 0, $x_{706}$-$x_{707}$, $x_{706}$-$x_{708}$, 0, $x_{709}$-$x_{710}$]. It can be seen that 9 sampling points corresponding to these 9 elements are the sampling point 702 to the sampling point 710. The nine elements represent cumulative changing degrees of sampling points corresponding thereto in respective signal segments.

Specifically, in an optional implementation, the above electronic device may determine the first feature sequence corresponding to the target sampling point by using the above target sampling point through the following method:

(1) Assuming that $x_t$ is the target sampling point, setting a time window as j+1, acquiring an amplitude of each sampling point within the window, and obtaining sequence $X_B$, such that $X_B = [x_t, x_{t+1}..., x_{t+j}]$.
(2) Computing a backward difference of adjacent elements in $X_B$, and obtaining sequence $M_{B1}$, such that $M_{B1} = [x_t - x_{t+1}, ..., x_{t+j-1} - x_{t+j}]$.
(3) Setting data less than zero in sequence $M_{B1}$ as zero, that is, $M_{B1}[M_{B1} < 0] = 0$, and obtaining sequence $M_{B2}$.
(4) Carrying out a forward accumulation and reconstruction operation on each original element in sequence $M_{B2}$, and obtaining first feature sequence $M_B$ corresponding to sampling point $x_t$. Specifically, if an element in sequence $M_{B2}$ is 0, the position is still 0 after reconstruction. If the position is not zero, forward accumulation is carried out until an element that is 0 is encountered. A value obtained through the forward accumulation is a value of the position after reconstruction.

That is, the above target sampling point is taken as a start point, and a first time window including (j+1) sampling points is determined. An amplitude difference between each sampling point except for a last sampling point in the above first time window and a sampling point after the sampling point is computed in sequence, and j differences are obtained. Numerical values less than 0 in these j differences are reset as 0, and a first sequence corresponding to the above target sampling point is obtained. A forward accumulation and reconstruction operation is carried out on elements in the first sequence corresponding to the above target sampling point in sequence, the above j elements are obtained, and the above j elements are taken as the first feature sequence corresponding to the target sampling point. The above accumulation and reconstruction operation includes: keeping a value of an element unchanged in a case that the value of the element is 0; and accumulating, in a case that the value of the element is not zero, the value of the element and a value of a previous element until an element having a value of 0 is encountered, and taking a value obtained through forward accumulation as a value after element reconstruction.

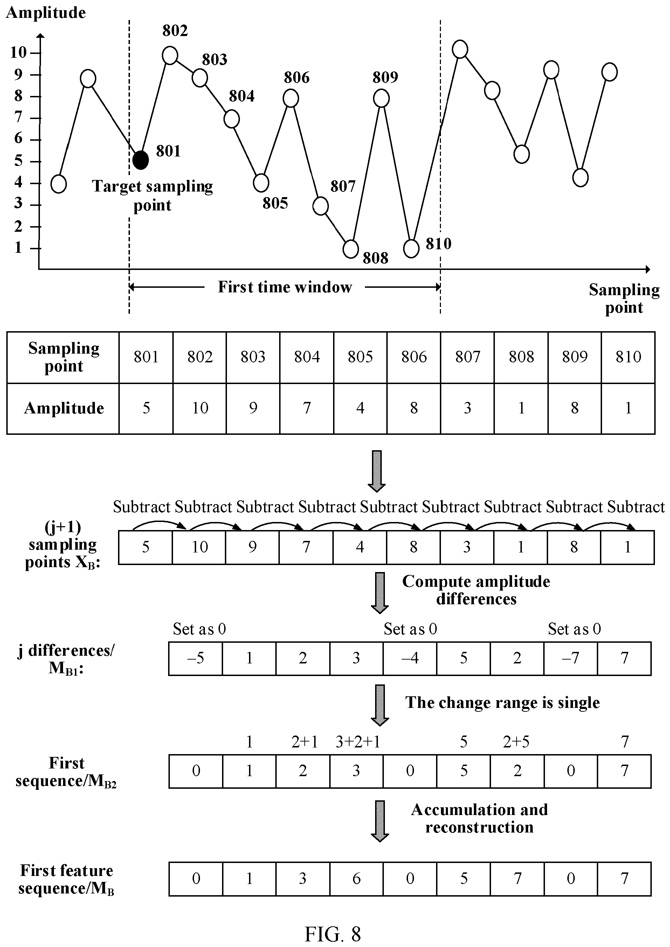

FIG. 8 is taken as an example for description. As shown in FIG. 8, the sampling point 801 in FIG. 8 is the above target sampling point. Assuming that j=9 herein, the 9 sampling points after sampling point 801, that is, the sampling point 802 to the sampling point 809 in FIG. 8, are the j sampling points corresponding to the above j elements. The sampling point 801 to the sampling point 810 are the above (j+1) sampling points, and the time window corresponding to the sampling point 801 to the sampling point 810 is the first time window.

Amplitudes of the sampling point 801 to the sampling point 810 are 5, 10, 9, 7, 4, 8, 3, 1, 8 and 1 respectively (it should be understood that the amplitude set herein is only for convenience of understanding by a reader and does not represent the amplitude of the signal to undergo noise removal in an actual scene, the same below). The amplitudes of the sampling point 801 to the sampling point 810 are arranged in sequence, that is sequence [5, 10, 9, 7, 4, 8, 3, 1, 8, 1] is obtained. Then, an amplitude difference between each of the above sampling point 801 to sampling point 810 and a sampling point after the sampling point is computed in sequence (a next term is subtracted from a previous term, computation is stopped if no sampling point exists after the sampling point 810), and 9 differences are obtained and are -5, 1, 2, 3, -4, 5, 2, -7 and 7. The 9 differences are the above j differences. The 9 differences are arranged in sequence, and new sequence [-5, 1, 2, 3, -4, 5, 2, -7, 7] is obtained. To simplify a change range (that is, only considering whether a segment of a stable and drastically-falling sampling point exists in the signal), the numeral values less than 0 in the new sequence is reset as 0, and first sequence [0, 1, 2, 3, 0, 5, 2, 0, 7] corresponding to the above target sampling point (that is, the sampling point 801) is obtained. Finally, a forward accumulation and reconstruction operation is carried out on the elements in the first sequence corresponding to the sampling point 801 in sequence, and a first feature sequence corresponding to the sampling point 801 is obtained. A first element in the first sequence is 0 such that the first element is still 0 after reconstruction. A second element is 1, and forward accumulation is carried out until 0 element is encountered, such that the element is still 1 after reconstruction. A third element is 2, and forward accumulation is carried out until 0 element is encountered, such that the element is still 2+1=3 after reconstruction. A fourth element is 3, and forward accumulation is carried out until 0 element is encountered, such that the element is still 3+2+1=6 after reconstruction. A fifth element is 0, such that the fifth element is still 0 after reconstruction. A sixth element is 5 after reconstruction. A 7th element is 2+5 after reconstruction. An eighth element is 0 after reconstruction. A ninth element is 7 after reconstruction. Thus, a first feature sequence finally obtained is [0, 1, 3, 6, 0, 5, 7, 0, 7].

S602: In a case that the numerical values in the first feature sequence corresponding to the above target sampling point are all less than the cumulative variation threshold corresponding to the target sampling point, the electronic device averages the amplitude of the target sampling point and the amplitudes of m sampling points around the target sampling point, obtains a target numerical value, and updates the amplitude of the target sampling point to the target numerical value.

The cumulative variation threshold corresponding to the above target sampling point is determined by using the first feature sequence corresponding to the above target sampling point and v first feature sequences corresponding to v sampling points before the target sampling point. Specifically, the specific action and the determination manner of the first feature sequence corresponding to any one of these v sampling points can be obtained with reference to the related description of the first feature sequence corresponding to the above target sampling point, and will not be repeated herein.

It should be understood that in a process that an electronic device removes noise from the above signal to undergo noise removal, the electronic device determines a sampling point of the signal to undergo noise removal as a target sampling point, and then determines the first feature sequence corresponding to the target sampling point through the method in the foregoing description. After processing the amplitude of the target sampling point, the electronic device takes the sampling point after the above target sampling point as a new target sampling point and acquires the first feature sequence corresponding to the new target sampling point through the same method. It is not difficult to understand that when the target sampling point changes, a first time window corresponding to the target sampling point will also move backwards (generally by one sampling point) accordingly. With FIG. 8 as an example, if a sampling point 800 having an amplitude of 9 exists before the sampling point 801, the sampling point 800 is a historical target sampling point before the electronic device is align with to remove noise from the sampling point 801 as the target sampling point, and a sequence of amplitudes of the sampling points in the first time window corresponding to the sampling point 800 is [9, 5, 10, 9, 7, 4, 8, 3, 1, 8]. Accordingly, according to the foregoing description, a first feature sequence corresponding to the sampling point 800 is [4, 0, 1, 3, 6, 0, 5, 7, 0] (herein, it is assumed that the sampling point 800 is a point on a wave peak or a wave trough).

That is, the electronic device takes each sampling point before the above target sampling point as the target sampling point and acquires the first feature sequences corresponding to these sampling points. Moreover, it can be seen from the first feature sequences corresponding to the sampling point 800 and the sampling point 811 that in the two first feature sequences corresponding to two adjacent sampling points, the remaining elements except for a last element in a first feature sequence corresponding to a sampling point after are all elements in the first feature sequence corresponding to the sampling point before.

In the method, after determining the first feature sequence corresponding to the above target sampling point, the electronic device determines a threshold, that is, the cumulative variation threshold corresponding to the above target sampling point, based on the first feature sequence corresponding to the above target sampling point and the first feature sequences corresponding to v sampling points before the above target sampling point. The cumulative variation threshold represents a distribution range of values of most elements in a sequence obtained by splicing the first feature sequence corresponding to the above target sampling point and v first feature sequences corresponding to v sampling points before the target sampling point. If a value of an element in the sequence is greater than or equal to the cumulative variation threshold, it indicates that the value of the element is significantly greater than values of the other elements in the sequence. That is, the element is an element on which an abrupt change occurs, which exactly conforms to the abrupt change performance of the QRS complex in the electrocardiography signal. Therefore, in the method, if the numerical values of the elements in the first feature sequence corresponding to the above target sampling point are all less than the cumulative variation threshold corresponding to the target sampling point, it indicates that the target sampling point is extremely likely to be a sampling point located in a stationary-segment signal in the signal to undergo noise removal. The electronic device may average an amplitude of the target sampling point and amplitudes of m sampling points around the target sampling point through an averaging filter method, obtain a target numerical value, and update the amplitude of the target sampling point to the target numerical value. Therefore, noise is removed from the target sampling point. Accordingly, if a numerical value of an element in the first feature sequence corresponding to the above target sampling point is greater than or equal to the cumulative variation threshold corresponding to the target sampling point, it indicates that the target sampling point is extremely likely to be a sampling point located in the QRS complex in the above signal to undergo noise removal. To protect the features of the QRS complex, the above electronic device does not change the amplitude of the above target sampling point.

A process of averaging the amplitudes of the m sampling points by the above electronic device may be summarized with the following formulas:

$$x_t^r = (x_{t-H} + \ldots + x_t +, \ldots, + x_{t+H})/(2H + 1),$$

where

$m = (2H + 1)$, $x_t^r$ is a new amplitude value set by the electronic device for target sampling point $x_t$ after noise is removed from target sampling point $x_t$, and m is equal to thirty percent of a sampling frequency of the above signal to undergo noise removal.

Specifically, the electronic device may splice the first feature sequence corresponding to the above target sampling point and the v first feature sequences corresponding to the v sampling points, and obtain a second sequence. The electronic device determines an outlier corresponding to the second sequence through a quartile method, and determines the outlier corresponding to the second sequence as the cumulative variation threshold corresponding to the target sampling point. Optionally, an outlier corresponding to the second sequence is an extreme outlier corresponding to the second sequence. The above process of determining an outlier corresponding to the second sequence through the quartile method may be generalized with the following formulas:

$$C_B = k1 * \text{percentile}(M_{B_H}, 75) - k2 * percentile(M_{B_H}, 25),$$

where

"*" represents multiplication operation, $C_B$ is the above cumulative variation threshold, $M_{BH} = [M_{B-v}, M_{B-v+1}, \ldots, M_B]$, $M_B$ is the first feature sequence corresponding to the above target sampling point, $M_{B-1}$ is a first feature sequence corresponding to a sampling point before the above target sampling point, and so on. Further, $M_{B_H}$ is a sequence obtained by unfolding, splicing and constructing a plurality of sequences of $M_{B-v}, M_{B-v+1}, \ldots, M_B$, etc., that is, the above second sequence. Further, percentile($M_{B_H}$, 75) represents a seventy-fifth quantile (upper quartile) of sequence $M_{B_H}$, and percentile($M_{B_H}$, 25) is a twenty-fifth quantile (lower quartile) of sequence $M_{B_H}$. Values of k1 and k2 are k1=4 and k2=3, or k1=2.5 and k2=1.5. In cases of k1=4 and k2=3, $C_B$ is an extreme outlier corresponding to the second sequence. In cases of k1=2.5 and k2=1.5, $C_B$ is a moderate outlier corresponding to the second sequence. It is not difficult to see that the electronic device adaptively updates the above cumulative variation threshold as the target sampling point is replaced.

The first feature sequence corresponding to the sampling point 800 and the first feature sequence corresponding to the sampling point 801 in the foregoing description are also taken as an example for description. The value of v mentioned above is 1. It can be known in combination with the foregoing description that the first feature sequence corresponding to the sampling point 800 is [4, 0, 1, 3, 6, 0, 5, 7, 0]. The first feature sequence corresponding to the sampling point 801 is [0, 1, 3, 6, 0, 5, 7, 0, 7]. That is, $M_B$=[0, 1, 3, 6, 0, 5, 7, 0, 7], and $M_{B-1}$=[4, 0, 1, 3, 6, 0, 5, 7, 0]. Then, $M_{BH}$ = [$M_{B-1}$, $M_B$]=[4, 0, 1, 3, 6, 0, 5, 7, 0]. Assuming that k1=4 and k2=3, $C_B$ = k1 * percentile($M_{B_H}$, 75) - k2 * percentile($M_{B_H}$, 25) =4 * 6.25+3 * 0=25. Since 25 is greater than the value of any one element in $M_{B_H}$, the electronic device determines the target sampling point 801 as the sampling point on the stationary segment. The electronic device updates the amplitude of the target sampling point 801, and specifically updates the amplitude of the target sampling point 801 to an average of a sum of amplitudes of m sampling points around the sampling point 801.

It can be known in combination with the foregoing description that since the amplitude of the sampling point in the QRS complex has a continuous abrupt change performance (or a sufficient number of continuous sampling points exist, and the amplitude of the sampling point remains sharply rising or falling), it can be understood that in cases of the sampling points in the QRS complex, elements having great numerical values are extremely likely to be generated in the first feature sequence corresponding to the sampling points due to cumulative variation of the amplitudes of the sampling points, and elements having numerical values greater than the cumulative variation threshold corresponding to the sampling points are extremely likely to be generated in the first feature sequence corresponding to the sampling points. Accordingly, in cases of sampling points in the stationary-segment signal, elements having great numerical values are less likely to be generated in the first feature sequence corresponding to these sampling points due to non-accumulability of amplitudes of the sampling points and too small variation ranges between the amplitudes. Elements having numerical values greater than the cumulative variation threshold corresponding to these sampling points are less likely to be found in the first feature sequence corresponding to these sampling points.

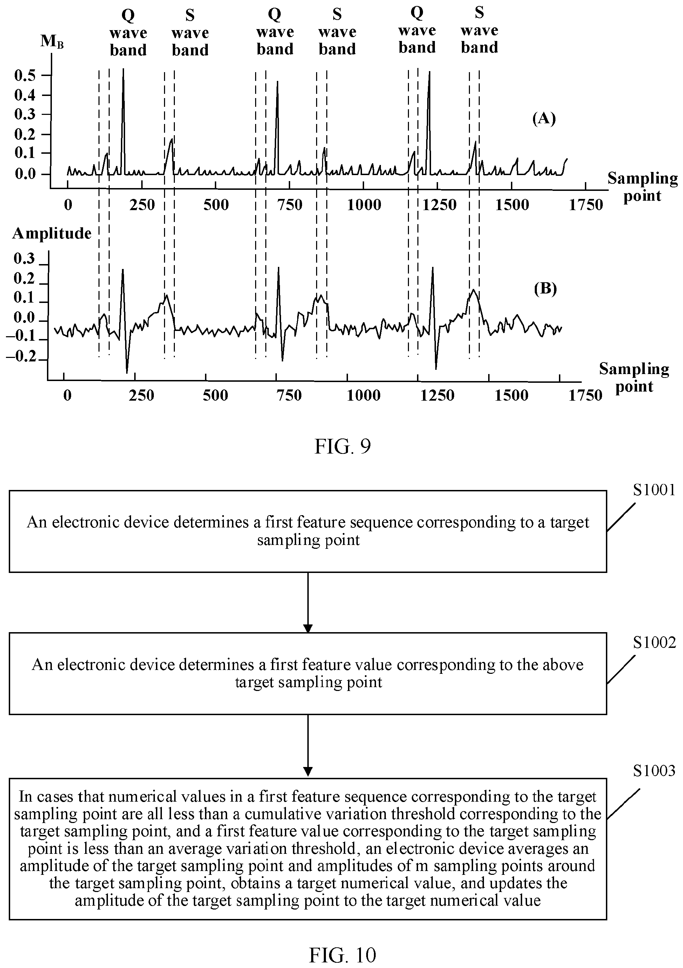

FIG. 9 shows an image of an initial signal without noise removal and an image of a sequence obtained by splicing first feature sequences corresponding to all sampling points in the initial signal. FIG. 9(B) shows an image of an initial signal without noise removal, which may be an image of the initial signal in the foregoing description. FIG. 9(A) is an image of first feature sequences corresponding to all sampling points in the initial signal. It can be known in combination with the foregoing description that in two first feature sequences corresponding to two adjacent sampling points, the remaining elements other than a last element in the first feature sequence corresponding to a sampling point after are elements in a first feature sequence corresponding to a sampling point before. Thus, a plurality of first feature sequences corresponding to a plurality of sampling points can be spliced, and a sequence is obtained (a sampling point corresponding to each element in the sequence may be a sampling point corresponding to the first feature sequence ending with the element). The image corresponding to the sequence is the image shown in FIG. 9(A). It can be seen that the image shown in FIG. 9(A) also shows obvious and sharp waves at positions where the Q wave, the R wave and the S wave appear in the initial signal. It is further verified that elements having numerical values greater than the cumulative variation threshold corresponding to these sampling points are extremely likely to appear in the first feature sequences corresponding to the sampling points on the Q wave, the R wave and the S wave in the initial signal. The electronic device can more accurately identify these sampling points without changing their amplitudes and specifically remove noise from the sampling points in the stationary-segment signal. Original features of the QRS complex in the electrocardiography signal can be retained, and noise is effectively removed from the electrocardiography signal on the premise that the reference value of the electrocardiography signal is guaranteed.

Based on the method for removing noise shown in FIG. 6, this application further provides another method for removing noise from a signal. This method identifies sampling points on waves having reference significance in the electrocardiography signals, particularly sampling points located on the R wave, during noise removal with reference to the continuous abrupt change performance of wave peaks having reference significance and the average variation degree of wave peaks having reference significance in the electrocardiography signals, thereby effectively avoiding suppression of sampling points on these waves during signal noise removal. The method is implemented, so that noise can be effectively removed from the electrocardiography signal on the premise that a reference value of the electrocardiography signal is further guaranteed.

Whether the signal point is a point on a signal wave having reference significance can be identified during noise removal, and whether to remove noise from the signal point can be determined. The method is implemented, so that noise can be removed from an electrocardiography signal without affecting a wave having reference significance in the electrocardiography signal. That is, noise can be effectively removed from the electrocardiography signal on the premise that a reference value of the electrocardiography signal is guaranteed. As shown in FIG. 10, the method provided in an embodiment of this application may include but is not limited to the following steps:

S1001: An electronic device determines a first feature sequence corresponding to a target sampling point.

The above electronic device may be the electronic device shown in FIG. 4 or the electronic device 100 in the foregoing description.

The above target sampling point is a sampling point in a signal to undergo noise removal. This signal to undergo noise removal may be a signal directly acquired by the above electronic device, or a signal acquired by another device and transmitted to the above electronic device.

Specifically, the above signal to undergo noise removal may be an electrocardiography signal. This electrocardiography signal may be a bioelectricity signal of a biological body surface extracted by the above electronic device by using an electrode. The acquired electrocardiography signal is composed of a plurality of sampling points ordered according to sampling time. In the electrocardiography signal, a value of the sampling point is an intensity or energy value of a bioelectricity signal of the body surface during acquisition. The plurality of sampling points are arranged in order of sampling time to form electrocardiography having a fluctuation degree.

Details of S1001 can be obtained with reference to the relevant description of S601 in FIG. 6, and will not be repeated herein. It should be noted that in an embodiment of this application, the electronic device may perform S1001 and S1002 in any order. That is, the electronic device may perform S1001 first and then perform S1002; the electronic device may perform S1002 first, and then perform S1001; or in some scenarios, the electronic device may simultaneously perform S1002 and S1001. This is not limited in this application.

S1002: An electronic device determines a first feature value corresponding to the above target sampling point.

The first feature value corresponding to the above target sampling point represents an average of absolute values of amplitude differences between every two adjacent sampling points in a time window including the target sampling point and including (k+1) sampling points.

In combination with the foregoing description, a QRS wave in the electrocardiography signal is generally the largest, most obvious and sharpest wave. This feature is mathematically referred to as singularity or abrupt change performance of the QRS wave and shown as an abrupt change in slope. Such a sharp change is more obviously embodied on the R wave of the QRS complex. Therefore, to enable the above electronic device to better determine whether the target sampling point is a point in the above QRS complex, the electronic device may determine a time window including (k+1) sampling points by taking the above target sampling point as a base point, and average absolute values of amplitude differences between every two adjacent sampling points in the time window, such that a first feature value corresponding to the above target sampling point is obtained.

It should be understood that unlike a cumulative variation degree, represented by the above first feature sequence, of a sampling point in a single change tendency, since the first feature value corresponding to the above target sampling point is obtained based on the absolute value of the amplitude difference between two sampling points, a change range between two sampling points is a number greater than 0 no matter how a change tendency of the above (k+1) sampling points changes. That is, no matter whether an amplitude of a sampling point in the time window including above (k+1) sampling points continuously rises, continuously falls, alternately rises or alternately falls, the first feature value corresponding to the above target sampling point is an average of total slope between every two adjacent sampling points of the above (k+1) sampling points. Then, in cases of sampling points on the QRS complex, especially on the R wave, slope (that is, an abrupt change degree) between each of points around these sampling points and a sampling point before the point and a sampling point after the point is extremely great, which means that the electronic device can easily determine whether the target sampling point is a point on the QRS wave, especially on the R wave, based on the first feature value corresponding to the above target sampling point, so as to determine whether noise is required to be removed from the target sampling point.

Optionally, in the above time window including the above target sampling point and including (k+1) sampling points, the above target sampling point is a last sampling point in the time window including the above (k+1) sampling points; or in the time window including the above target sampling point and including the (k+1) sampling points, at least one sampling point exists before the above target sampling point and at least one sampling point exists after the target sampling point.

That is, after currently acquiring a latest sampling point signal, the above electronic device may acquire k sampling points before the sampling point, and compute an average of absolute values of amplitude differences between every two adjacent sampling points of the (k+1) sampling points ending with the sampling point as the first feature value corresponding to the sampling point. In this way, the electronic device can process the just-acquired sampling point without delay, and more efficiently process the target sampling point. Alternatively, after currently acquiring a signal of the above latest one sampling point, the above electronic device may not immediately process the sampling point, but acquires at least one sampling point before the sampling point and at least one sampling point before the sampling point, which are k sampling points in total, with the sampling point as a base point after continuing acquiring signals of several sampling points after the sampling point, determines the k sampling points and the sampling point as the above (k+1) sampling points, and compute the average of the absolute values of the amplitude differences between every two adjacent sampling points of these (k+1) sampling points as the first feature value corresponding to the sampling point. In this way, although the electronic device cannot process the just-acquired sampling point, by taking the above target sampling point as the sampling point at a middle position of the above (k+1) sampling points, the computed first feature value corresponding to the above target sampling point can more realistically reflect the change tendency of the above target sampling point, and whether the above target sampling point is a point on the QRS complex can be more accurately determined.

Specifically, in an optional implementation, the above electronic device may determine the first feature value corresponding to the target sampling point by using the above target sampling point through the following method:

(1) Assuming that $x_t$ is a target sampling point, setting a time window as k+1, obtaining an amplitude of each sampling point within the window, and obtaining sequence $X_A$, such that $X_A = [x_{t-a},...,x_{t-1},x_t,x_{t+1},...,x_{t+b}]$, and k=a+b.

(2) Computing average $X_A$ of absolute values of differences of sequence $m^A$, such that $m^A$ is the first feature value corresponding to target sampling point $x_t$, and

$$m^A = \frac{\sum_{q=t-k}^{q=t+k}(|x_q - x_{q-1}|)}{2k+1}.$$

That is, the electronic device may determine a second time window including (k+1) sampling points. In the second time window, n sampling points exist before the above target sampling point, and (k-n) sampling points exist after the above target sampling point. Then, the electronic device may compute an absolute value of a difference between each sampling point and a sampling point before the sampling point in the second time window in sequence, obtain absolute values of k differences, and take an average of the absolute values of the k differences as the first feature value corresponding to the target sampling point.

S1003: In a case that the numerical values in the first feature sequence corresponding to the above target sampling point are all less than the cumulative variation threshold corresponding to the target sampling point, and the first feature value corresponding to the above target sampling point is less than the average variation threshold, the electronic device averages an amplitude of the target sampling point and amplitudes of m sampling points around the target sampling point, obtains a target numerical value, and updates the amplitude of the target sampling point to the target numerical value.

The cumulative variation threshold corresponding to the above target sampling point is determined by using the first feature sequence corresponding to the above target sampling point and v first feature sequences corresponding to v sampling points before the target sampling point.

Specifically, the details of "the numerical values in the first feature sequence corresponding to the above target sampling point are all less than the cumulative variation threshold corresponding to the target sampling point" and "the electronic device averages an amplitude of the target sampling point and amplitudes of m sampling points around the target sampling point by , obtains a target numerical value, and updates the amplitude of the target sampling point to the target numerical value" in S 1003 can be obtained with reference to the related description of S602 in FIG. 6, and will not be repeated herein.

The average variation threshold corresponding to the above target sampling point is determined by using the first feature value corresponding to the above target sampling point and i first feature sequences corresponding to i sampling points before the target sampling point. Specifically, the specific action and the determination manner of the first feature value corresponding to any one of these i sampling points can be obtained with reference to the related description of the first feature value corresponding to the above target sampling point, and will not be repeated herein.

Similarly, in a process of removing noise from the above signal to undergo noise removal by the above electronic device, the electronic device determines a sampling point of the above signal to undergo noise removal as a target sampling point, and then determines the first feature value corresponding to the target sampling point through a method in the foregoing description. After processing the amplitude of the target sampling point, the electronic device takes the sampling point after the above target sampling point as a new target sampling point and acquires the first feature value corresponding to the new target sampling point in the same manner. It is not difficult to understand that when the target sampling point changes, a second time window corresponding to the target sampling point will also move backwards (generally by one sampling point) accordingly. With FIG. 8 mentioned above as an example, if a sampling point 800 having an amplitude of 100 exists before the sampling point 801, the sampling point 800 is a historical target sampling point before the electronic device is align with to remove noise from the sampling point 801 as the target sampling point, and the first feature value corresponding to the sampling point 801 is (5+1+2+3+4+5+2+7+7)/10=3.6. The first feature value corresponding to the sampling point 800 is (4+5+1+2+3+4+5+2+7)/10=3.3.

That is, the electronic device takes each sampling point before the above target sampling point as the target sampling point and acquires the first feature values corresponding to these sampling points. In the method, after determining the first feature value corresponding to the above target sampling point, the electronic device determines a threshold, that is, the average variation threshold corresponding to the above target sampling point, based on the first feature value corresponding to the above target sampling point and the first feature values corresponding to i sampling points before the above target sampling point. The cumulative variation threshold represents a distribution range of values of most elements in a sequence obtained by splicing the first feature value corresponding to the above target sampling point and v first feature values corresponding to i sampling points before the above target sampling point. If an element in the sequence has a value greater than or equal to the average variation threshold, it indicates that the value of the element is significantly greater than values of the other elements in the sequence. That is, the element is an element on which an abrupt change occurs, which exactly conforms to the abrupt change performance of the QRS complex, especially the abrupt change performance of slope of the sampling point on the R wave, in the electrocardiography signal. Therefore, in the method, if the first feature value corresponding to the above target sampling point are all less than the average variation threshold corresponding to the target sampling point, it indicates that the target sampling point is extremely likely to be a sampling point located in a stationary-segment signal in the above signal to undergo noise removal. The electronic device may average an amplitude of the target sampling point and amplitudes of m sampling points around the target sampling point through an averaging filter method, obtain a target numerical value, and update the amplitude of the target sampling point to the target numerical value. Therefore, noise is removed from the target sampling point. Accordingly, if the first feature value corresponding to the above target sampling point is greater than or equal to the average variation threshold corresponding to the target sampling point, it indicates that the target sampling point is extremely likely to be a sampling point located in the QRS complex in the above signal to undergo noise removal. To protect the features of the QRS complex, the electronic device does not change the amplitude of the target sampling point.

Specifically, the electronic device may splice the first feature value corresponding to the above target sampling point and the i first feature values corresponding to the i sampling points, and obtain a third sequence. The electronic device determines an outlier corresponding to the third sequence through a quartile method, and determines the outlier corresponding to the third sequence as the cumulative variation threshold corresponding to the target sampling point. Optionally, an outlier corresponding to the third sequence is an extreme outlier of the second sequence. The above process of determining an outlier corresponding to the third sequence through the quartile method may be generalized with the following formulas:

$$C_A = k1 * \mathrm{percentile}\big(M_{A_H}, 75\big) - k2 * percentile\big(M_{A_H}, 25\big),$$

where
"*" represents multiplication operation, $C_A$ is the cumulative variation threshold,

$M_{A_H} = [m^A_{t-i}, m^A_{t-i+1}, m^A_{t-i+2}, \ldots, m^A_t]$ , $M_A$ is the first feature value corresponding to the target sampling point, $M_{A-1}$ is a first feature value corresponding to a sampling point before the above target sampling point, and so on. Further,

$M_{AH}$ is the above third sequence. Further, percentile($M_{AH}$, 75) represents a seventy-fifth quantile (upper quartile) of sequence $M_{AH}$, and percentile($M_{AH}$, 25) is a twenty-fifth quantile (lower quartile) of sequence $M_{BAH}$. Values of $k1$ and $k2$ are $k1$=4 and $k2$=3, or $k1$=2.5 and $k2$=1.5. In cases of $k1$=4 and $k2$=3, $C_A$ is an extreme outlier corresponding to the third sequence. In cases of $k1$=2.5 and $k2$=1.5, $C_A$ is a moderate outlier corresponding to the third sequence. It is not difficult to see that the electronic device can also adaptively update the above average variation threshold as the target sampling point is replaced.

The first feature sequence corresponding to the sampling point 800 and the first feature sequence corresponding to the sampling point 801 in the foregoing description are also taken as an example for description. For convenience of description, it is assumed herein that the value of v mentioned above is 1, i is 7, and the sampling point 801 is the above target sampling point, and the third sequence constructed based on the sampling point 801 is [6, 2.7, 4, 3.5, 5, 3.3, 3.6]. It can be known in combination with the foregoing description that a first feature sequence corresponding to the sampling point 801 is [4, 0, 1, 3, 6, 0, 5, 7, 0], and the sampling point 801 corresponds to $C_B$ =28. The sampling point 801 corresponds to $C_A$ = 4 * 5 - 3 * 3.3 = 10.1, and $m^A$=3.6.

The values of the elements in first feature sequence [4, 0, 1, 3, 6, 0, 5, 7, 0] corresponding to the sampling point 801 are all less than 25. The electronic device makes first feature value $m^A$ corresponding to the target sampling point 801 less than $C_A$, determines the target sampling point 801 as a sampling point in the Q-stationary-segment signal, and updates the amplitude of the target sampling point 801 to be the average of the sum of the amplitudes of the m sampling points around the sampling point 801.

It should be understood that in cases of some abnormal electrocardiography signals, a direction of an R wave in the QRS complex in the signal may be reversed. For example, R waves in QRS complexes in electrocardiography signals of some people suffering from heart diseases may be inverted due to myocardial ischemia of the patients. In this case, rising and falling tendencies of the sampling point in the R wave are reversed. Change tendencies between wave peaks and wave troughs of the R wave and PR and ST segments are reversed. In this case, if a position of the target sampling point is analyzed only by constructing the first feature sequence corresponding to the target sampling point, an erroneous conclusion is extremely likely to be acquired. Moreover, the amplitude of the target sampling point is extremely likely to be processed through an improper method in a noise removal process. However, the first feature value corresponding to the target sampling point focuses on only a change range of the amplitude of the sampling point and does not focus on a change tendency of the amplitude of the sampling point. If a region where the target sampling point is located is determined by using the first feature value corresponding to the target sampling point and the first feature sequence corresponding to the target sampling point, determination of a position of a sampling point on an abnormal electrocardiography signal can be extremely likely to be reduced, whether the target signal point is a point on the QRS complex is determined, and noise is removed from the stationary-segment signal in the signal on the premise that features of the Q wave and the S wave are retained. In addition, by using the first feature value corresponding to the target sampling point, the electronic device can more quickly and accurately determine whether a normal electrocardiography signal is a sampling point on the R wave.

In an optional implementation, the first feature sequence and the first feature value corresponding to the above target sampling point may correspond to two data features FB and FA of the target sampling point respectively. If first feature value $m^A$ corresponding to the target sampling(point is greater than or equal to average variation threshold $C^A$ corresponding to the target sampling point, a feature of the target sampling point is $F_A$ = 1, and otherwise, $F_A$ = 0. If at least one element in first feature sequence $M_B$ corresponding to the above target sampling point is greater than or equal to cumulative variation threshold $C_B$ corresponding to the target sampling point, data of the above target sampling point is $F_B$ = 1, and otherwise, $F_B$ = 0. It can be known in combination with the foregoing description that in a case of the amplitude corresponding to the target sampling point, if $F_A$ = 0 and $F_B$ = 0, it indicates that the target sampling point is the sampling point in the stationary-segment signal (suppressed region), the amplitude of the target sampling point is updated to an average of sums of the amplitudes of the m sampling points around the target sampling point. If a value of at least one data feature in $F_A$ and $F_B$ is 1, it indicates that the target sampling point is a sampling point in the QRS complex (non-suppressed region), and the amplitude of the target sampling point is kept unchanged. Details are shown in following Table 1:

**Table 1**

| Values of data features | Conditions | Method for processing amplitude of target sampling point |
|---|---|---|
| $F_A$ = 0 and $F_B$ = 0 | $m^A$ is less than $C^A$, and the values of all elements in $M_B$ are less than $C_B$ (the target sampling point is located in a stationary-segment signal) | Update the amplitude as an average of sums of amplitudes of m sampling points around a target sampling point |

(continued)

| Values of data features | Conditions | Method for processing amplitude of target sampling point |
|---|---|---|
| $F_A = 0$ and $F_B = 1$ | $m^A$ is less than $C^A$, and a value of at least one element in $M_B$ is greater than or equal to $C_B$ (the target sampling point is located on a Q wave or an S wave) | Keep unchanged |
| $F_A = 1$ and $F_B = 1$ | $m^A$ is greater than or equal to $C^A$, and a value of at least one element in $M_B$ is greater than or equal to $C_B$ (the target sampling point is located on a QRS complex, and extremely likely on an R wave) | Keep unchanged |

Similarly, the amplitude of the sampling point in the QRS complex has a continuous abrupt change performance. Especially, the abrupt change performance of the amplitude of the sampling points on the R wave is extremely obvious. It can be understood that first feature values corresponding to sampling points on the QRS complex are extremely likely to be greater than an average variation threshold corresponding to these sampling points due to drastic changes in amplitudes of the sampling points. Accordingly, first feature values corresponding to sampling points in the stationary-segment signal are less likely to be greater numerical values due to too little variation ranges between amplitudes of the sampling points, and are less likely to be greater than the average variation threshold of the sampling points.

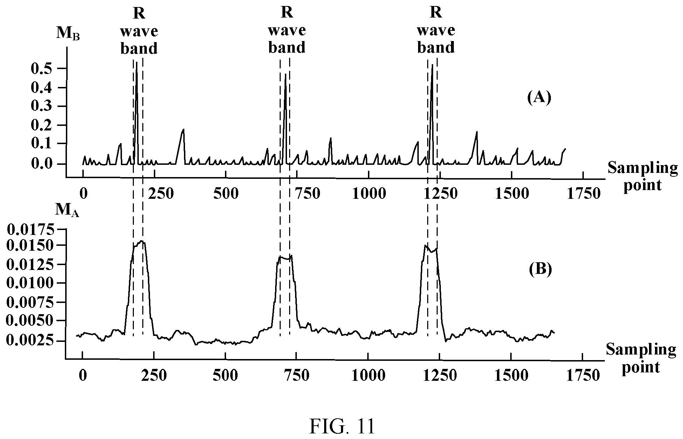

FIG. 11 shows an image of an initial signal without noise removal and an image of first feature values corresponding to all sampling points in the initial signal. FIG. 11(B) shows an image of an initial signal without noise removal, which may be an image of the initial signal in the foregoing description. FIG. 11(A) is an image of the first feature values corresponding to all sampling points in the initial signal. It can be seen that the image shown in FIG. 11(A) also shows obvious and great-amplitude waves at the corresponding positions where the R waves appear in the initial signal. It is further verified that first feature values corresponding to the sampling points of the R wave in the initial signal are extremely likely to be greater than the average variation threshold corresponding to these sampling points. The electronic device can more accurately identify these sampling points without changing amplitudes and specifically remove noise from the sampling points in the stationary-segment signal. Original features of the R wave of the QRS complex in the electrocardiography signal can be retained, and noise is effectively removed from the electrocardiography signal on the premise that the reference value of the electrocardiography signal is guaranteed.

An embodiment of this application further provides an electronic device. This electronic device includes: one or more processors and a memory.

The memory is coupled to the one or more processors. This memory is configured to store computer program code. The computer program code includes computer instructions. The one or more processors invoke the computer instructions to enable the electronic device to perform the method shown in the above embodiment.

As used in the above embodiments, based on the context, the term "in a case..." can be interpreted as a meaning of "if...", "after...", "in response to determining... ", or "in response to detecting...". Similarly, based on the context, the phrase "if determining..." or "if detecting... (a stated condition or event)" can be interpreted as a meaning of "if determining..." or "in response to determining... ", or "if detecting (a stated condition or event)" or "in response to detecting (a stated condition or event)".

All or some of the above embodiments may be implemented by using software, hardware, firmware, or their combinations. In a case that software is used for implementation, implementation may be entirely or partially carried out in the form of a computer program product. The computer program product includes one or more computer instructions. When the computer program instructions are loaded and performed on a computer, all or some of flows or functions according to embodiments of this application are generated. The computer may be a general-purpose computer, a dedicated computer, a computer network, or another programmable apparatus. The computer instructions may be stored in a computer-readable storage medium or may be transmitted from a computer-readable storage medium to another computer-readable storage medium. For example, the computer instructions may be transmitted from a website, computer, server, or data center to another website, computer, server, or data center in a wired (for example, coaxial-cable, optical-fiber, or digital-subscriber-line) or wireless (for example, infrared, radio, or microwave) manner. The computer-readable storage medium may be any available medium that can be accessed by the computer, or a data storage device, such as a server or a data center in which one or more usable media are integrated. The usable medium may be a magnetic medium (such as a floppy disk, a hard disk, or a magnetic tape), an optical medium (such as a DVD), a semiconductor medium (such as a solid-state drive), etc.

Those of ordinary skill in the art can understand that all or some flows of the method in the above embodiments may be implemented by using a computer program instructing relevant hardware. The program may be stored in a computer-readable storage medium. When the program is performed, flows of all method embodiments mentioned above may be included. The storage medium includes: any medium that can store program code, such as a read only memory (ROM), a random access memory (RAM), a magnetic disk, and an optical disk.

**Claims**

1. A method for removing noise from an electrocardiography signal, the method performed by an electronic device comprising a processor, the method comprising:

   determining (S601) a first feature sequence corresponding to a target sampling point, wherein the first feature sequence corresponding to the target sampling point comprises j elements, the j elements correspond to, in a one-to-one manner, j sampling points in a signal to undergo noise removal, an element corresponding to any sampling point of the j sampling points represent a sum of rising or falling ranges of all sampling points between the any sampling point and a first sampling point, a falling or rising range of the any sampling point of the j sampling points is equal to a difference between an amplitude of the any sampling point and an amplitude of a sampling point after the any sampling point, and the first sampling point is a sampling point that is located before the any sampling point, has an opposite change tendency relative to the any sampling point, and is closest to the any sampling point; and

   averaging (S602) an amplitude of the target sampling point and amplitudes of m sampling points around the target sampling point, and obtaining a target numerical value in a case that numerical values in the first feature sequence corresponding to the target sampling point are all less than a cumulative variation threshold corresponding to the target sampling point, and updating the amplitude of the target sampling point to the target numerical value, wherein the cumulative variation threshold corresponding to the target sampling point is determined by using the first feature sequence corresponding to the target sampling point and v first feature sequences corresponding to v sampling points before the target sampling point, where v = 1 and m is equal to thirty percent of a sampling frequency of the sampling points to undergo noise removal.

2. The method according to claim 1, wherein
   before the averaging an amplitude of the target sampling point and amplitudes of m sampling points around the target sampling point, and obtaining a target numerical value, the method further comprises:

   determining a first feature value corresponding to the target sampling point, wherein the first feature value corresponding to the target sampling point represents an average of absolute values of amplitude differences between every two adjacent sampling points in a time window comprising the target sampling point and comprising (k+1) sampling points; and
   the averaging an amplitude of the target sampling point and amplitudes of m sampling points around the target sampling point, and obtaining a target numerical value comprise:
   averaging the amplitude of the target sampling point and the amplitudes of the m sampling points around the target sampling point, and obtaining the target numerical value in a case that the numerical values in the first feature sequence corresponding to the target sampling point are all less than the cumulative variation threshold corresponding to the target sampling point, and the first feature value corresponding to the target sampling point is less than an average variation threshold, wherein the average variation threshold corresponding to the target sampling point is determined by using the first feature value corresponding to the target sampling point and first feature values corresponding to i sampling points; and in the signal to undergo noise removal, the i sampling points are i sampling points before the target sampling point.

3. The method according to claim 1 or 2, the method further comprises:

   keeping the amplitude of the target sampling point unchanged in a case that at least one numerical value in the first feature sequence corresponding to the target sampling point is greater than or equal to the cumulative variation threshold corresponding to the target sampling point; and/or
   keeping the amplitude of the target sampling point unchanged in a case that the first feature value corresponding to the target sampling point is greater than or equal to the average variation threshold.

4. The method according to any one of claims 1 to 3, wherein before the determining a first feature sequence corresponding to a target sampling point, the method further comprises:
   high-pass-filtering and low-pass-filtering an initial signal, and obtaining the signal to undergo noise removal, wherein the initial signal is an electrical signal that represents a heart rhythm of a user and is acquired by an electronic device.

5. The method according to any one of claims 2 to 4, wherein in the time window comprising the target sampling point and comprising the (k+1) sampling points, the target sampling point is a last sampling point in the time window comprising the (k+1) sampling points; or in the time window comprising the target sampling point and comprising the (k+1) sampling points, at least one sampling point exists before the target sampling point, and at least one sampling point exists after the target sampling point.

6. The method according to any one of claims 1 to 5, wherein the determining a first feature sequence corresponding to a target sampling point comprises:

taking the target sampling point as a start point, and determining a first time window comprising (j+1) sampling points;

computing an amplitude difference between each sampling point except for a last sampling point in the first time window and a sampling point after the sampling point in sequence, and obtaining j differences;

resetting numerical values less than 0 in the j differences as 0, and obtaining a first sequence corresponding to the target sampling point; and

carrying out a forward accumulation and reconstruction operation on elements in the first sequence corresponding to the target sampling point in sequence, obtaining the j elements, and taking the j elements as the first feature sequence corresponding to the target sampling point, wherein the accumulation and reconstruction operation comprises: keeping a value of an element unchanged in a case that the value of the element is 0; and accumulating, in a case that the value of the element is not zero, the value of the element and a value of a previous element until an element having a value of 0 is encountered, and taking a value obtained through forward accumulation as a value after element reconstruction.

7. The method according to any one of claims 2 to 6, wherein the determining a first feature value corresponding to the target sampling point comprises:

determining a second time window comprising (k+1) sampling points, wherein in the second time window, n sampling points exist before the target sampling point, and (k-n) sampling points exist after the target sampling point; and

computing an absolute value of a difference between each sampling point and a sampling point before the sampling point in the second time window in sequence, obtaining absolute values of k differences, and taking an average of the absolute values of the k differences as the first feature value corresponding to the target sampling point.

8. The method according to any one of claims 1 to 7, wherein after the determining a first feature sequence corresponding to a target sampling point, the method further comprises:

splicing the first feature sequence corresponding to the target sampling point and the v first feature sequences corresponding to the v sampling points, obtaining a second sequence,

determining an outlier corresponding to the second sequence through a quartile method, and determining the outlier corresponding to the second sequence as the cumulative variation threshold corresponding to the target sampling point.

9. The method according to any one of claims 2 to 8, wherein after the determining a first feature value corresponding to the target sampling point, the method further comprises:

constructing a third sequence by using the first feature value corresponding to the target sampling point and i first feature values corresponding to the i sampling points,

determining an outlier corresponding to the third sequence through the quartile method, and determining the outlier corresponding to the third sequence as the average variation threshold corresponding to the target sampling point.

10. The method according to any one of claims 8 or 9, wherein the cumulative variation threshold corresponding to the target sampling point is an extreme outlier corresponding to the second sequence determined through the quartile method, and/or the average variation threshold corresponding to the target sampling point is an extreme outlier corresponding to the third sequence determined through the quartile method.

11. An electronic device (100) wherein the electronic device comprises: one or more processors, a memory (121), and a display (171) and the memory (121) is coupled to the one or more processors (110), the memory (121) is configured to store computer program code, the computer program code comprises computer instructions, and the one or more processors invoke the computer instructions to enable the electronic device to perform the method according to any one of claims 1 to 10.

12. A computer-readable storage medium (121), comprising instructions, wherein when the instructions are run on an electronic device (100) the electronic device (100) is enabled to perform the method according to any one of claims 1 to 10.

**Patentansprüche**

1. Verfahren zur Entfernung von Störsignalen aus einem Elektrokardiogramm-Signal, wobei das Verfahren von einem elektronischen Gerät mit einem Prozessor durchgeführt wird und folgendes umfasst:

   Bestimmen (S601) einer ersten Merkmalssequenz, die einem Zielabtastpunkt entspricht, wobei die erste Merkmalssequenz, die dem Zielabtastpunkt entspricht, j Elemente umfasst, wobei die j Elemente jeweils einem der j Abtastpunkte in einem Signal zur Rauschunterdrückung eindeutig zugeordnet werden. Ein Element, das einem beliebigen Abtastpunkt der j Abtastpunkte entspricht, stellt die Summe der steigenden oder fallenden Bereiche aller Abtastpunkte zwischen diesem beliebigen Abtastpunkt und einem ersten Abtastpunkt dar. Der fallende oder steigende Bereich des jeweiligen Abtastpunktes der j Abtastpunkte entspricht der Differenz zwischen der Amplitude des jeweiligen Abtastpunktes und der Amplitude eines darauf folgenden Abtastpunktes. Der erste Abtastpunkt ist ein Abtastpunkt, der vor dem jeweiligen Abtastpunkt liegt, eine entgegengesetzte Änderungstendenz relativ zum jeweiligen Abtastpunkt aufweist und am nächsten zum jeweiligen Abtastpunkt positioniert ist; und
   Mittelung (S602) einer Amplitude des Zielabtastpunktes und der Amplituden von m Abtastpunkten um den Zielabtastpunkt herum, Ermitteln eines Zielwertes, vorausgesetzt, die Werte in der ersten Merkmalssequenz, die dem Zielabtastpunkt entsprechen, sind alle kleiner als ein kumulativer Variationsschwellenwert, der für den Zielabtastpunkt gilt, und Aktualisierung der Amplitude des Zielabtastpunkts auf den Zielwert, wobei der kumulative Variationsschwellenwert für den Zielabtastpunkt unter Verwendung der ersten Merkmalssequenz des Zielabtastpunktes und der v ersten Merkmalssequenzen für v Abtastpunkte vor dem Zielabtastpunkt bestimmt wird, wobei v = 1 und m dreißig Prozent der Abtastrate der abzutastenden Punkte zur Rauschunterdrückung entspricht.

2. Das Verfahren nach Anspruch 1, wobei
   Bevor die Amplitude des Zielabtastpunktes und die Amplituden von m Abtastpunkten um den Zielabtastpunkt herum gemittelt und ein Zielwert ermittelt werden, umfasst das Verfahren weiterhin:

   Bestimmen eines ersten Merkmalswerts, der dem Zielabtastpunkt entspricht, wobei der erste Merkmalswert des Zielabtastpunktes den Mittelwert der Absolutwerte der Amplitudendifferenzen zwischen jeweils zwei benachbarten Abtastpunkten in einem Zeitfenster mit dem Zielabtastpunkt und insgesamt (k+1) Abtastpunkten darstellt; und
   Das Mitteln der Amplitude des Zielabtastpunktes und der Amplituden der m Abtastpunkte um den Zielabtastpunkt sowie das Ermitteln eines Zielwertes umfasst:
   Durchschnittsbildung der Amplitude des Zielabtastpunkts und der Amplituden der m Abtastpunkte um den Zielabtastpunkt herum und Ermittlung des Zielnumerischen Werts für den Fall, dass die numerischen Werte in der ersten Merkmalssequenz, die dem Zielabtastpunkt entsprechen, alle kleiner als der kumulative Änderungsschwellenwert sind, der dem Zielabtastpunkt entspricht, und der erste Merkmalswert, der dem Zielabtastpunkt entspricht, kleiner als ein durchschnittlicher Änderungsschwellenwert ist, wobei der durchschnittliche Änderungsschwellenwert, der dem Zielabtastpunkt entspricht, unter Verwendung des ersten Merkmalswerts des Zielabtastpunkts und der ersten Merkmalswerte der i Abtastpunkte bestimmt wird; und im Signal, das einer Rauschunterdrückung unterzogen werden soll, sind die i Abtastpunkte die i Abtastpunkte vor dem Zielabtastpunkt.

3. Verfahren nach Anspruch 1 oder 2, wobei das Verfahren weiterhin umfasst:

   Die Amplitude des Zielabtastpunkts bleibt unverändert, falls mindestens ein numerischer Wert in der ersten Merkmalssequenz, der dem Zielabtastpunkt entspricht, größer oder gleich dem kumulativen Änderungsschwellenwert ist, der dem Zielabtastpunkt entspricht; und/oder

Die Amplitude des Zielabtastpunkts bleibt unverändert, wenn der erste Merkmalswert, der dem Zielabtastpunkt entspricht, größer oder gleich dem durchschnittlichen Änderungsschwellenwert ist.

4. Verfahren nach einem der Ansprüche 1 bis 3, wobei vor der Bestimmung einer ersten Merkmalssequenz, die einem Zielabtastpunkt entspricht, das Verfahren weiterhin umfasst:

Hochpass- und Tiefpassfilterung eines Eingangssignals und Erzeugung des Signals zur Rauschunterdrückung, wobei das Eingangssignal ein elektrisches Signal ist, das den Herzrhythmus eines Benutzers darstellt und von einem elektronischen Gerät erfasst wird.

5. Verfahren nach einem der Ansprüche 2 bis 4, wobei im Zeitfenster, das den Zielabtastpunkt und die (k+1) Abtastpunkte umfasst, der Zielabtastpunkt der letzte Abtastpunkt im Zeitfenster mit den (k+1) Abtastpunkten ist; oder im Zeitfenster, das den Zielabtastpunkt und die (k+1) Abtastpunkte umfasst, mindestens ein Abtastpunkt vor dem Zielabtastpunkt und mindestens ein Abtastpunkt nach dem Zielabtastpunkt existiert.

6. Verfahren nach einem der Ansprüche 1 bis 5, wobei die Bestimmung einer ersten Merkmalssequenz, die einem Zielabtastpunkt entspricht, umfasst:

Den Zielabtastpunkt als Startpunkt nehmen und ein erstes Zeitfenster mit (j+1) Abtastpunkten bestimmen; Berechnen einer Amplitudendifferenz zwischen jedem Abtastpunkt mit Ausnahme des letzten Abtastpunkts im ersten Zeitfenster und einem nachfolgenden Abtastpunkt in der Sequenz und Erhalten von j Differenzen; Zurücksetzen von Zahlenwerten kleiner als 0 in den j-Differenzen auf 0 und Erhalten einer ersten Sequenz, die dem Zielabtastpunkt entspricht; und

Durchführung einer Vorwärtsakkumulations- und Rekonstruktionsoperation an den Elementen der ersten Sequenz, die dem Zielabtastpunkt entspricht, der Reihe nach, Erhalt der j Elemente und Festlegung dieser j Elemente als die erste Merkmalssequenz, die dem Zielabtastpunkt entspricht, wobei die Akkumulations- und Rekonstruktionsoperation Folgendes umfasst: Den Wert eines Elements beibehalten, sofern der Wert 0 ist; und im Falle eines Wertes ungleich 0, den Wert des Elements und den Wert des vorherigen Elements akkumulieren, bis ein Element mit Wert 0 gefunden wird, und den durch Vorwärtsakkumulation erhaltenen Wert als Wert nach der Rekonstruktion festlegen.

7. Verfahren nach einem der Ansprüche 2 bis 6, wobei das Bestimmen eines ersten Merkmalswertes, der dem Zielabtastpunkt entspricht, Folgendes umfasst:

Bestimmen eines zweiten Zeitfensters, das (k+1) Abtastpunkte umfasst, wobei im zweiten Zeitfenster n Abtastpunkte vor dem Zielabtastpunkt und (k-n) Abtastpunkte nach dem Zielabtastpunkt vorhanden sind; und Berechnen eines Absolutwertes einer Differenz zwischen jedem Abtastpunkt und einem vorherigen Abtastpunkt im zweiten Zeitfenster der Reihe nach, Erhalten der Absolutwerte von k Differenzen und Nehmen des Mittelwerts der Absolutwerte der k Differenzen als den ersten Merkmalswert, der dem Zielabtastpunkt entspricht.

8. Verfahren nach einem der Ansprüche 1 bis 7, wobei nach dem Bestimmen einer ersten Merkmalssequenz, die einem Zielabtastpunkt entspricht, das Verfahren ferner Folgendes umfasst:

Verknüpfen der ersten Merkmalssequenz, die dem Zielabtastpunkt entspricht, und der v ersten Merkmalssequenzen, die den v Abtastpunkten entsprechen, und Erhalten einer zweiten Sequenz, Bestimmen eines Ausreißers, der der zweiten Sequenz mittels Quartil-Methode entspricht, und Festlegen des Ausreißers der zweiten Sequenz als Schwellenwert für die kumulative Variation, der dem Zielabtastpunkt entspricht.

9. Verfahren nach einem der Ansprüche 2 bis 8, wobei nach dem Bestimmen eines ersten Merkmalswertes, der dem Zielabtastpunkt entspricht, das Verfahren ferner Folgendes umfasst:

Konstruktion einer dritten Sequenz durch Verwendung des ersten Merkmalswerts, der dem Zielabtastpunkt entspricht, und i ersten Merkmalswerten, die den i Abtastpunkten entsprechen, Bestimmung eines Ausreißers, der der dritten Sequenz durch die Quartil-Methode entspricht, und Festlegung des Ausreißers der dritten Sequenz als Durchschnittsabweichungsschwelle, die dem Zielabtastpunkt entspricht.

10. Verfahren gemäß einem der Ansprüche 8 oder 9, wobei die kumulative Abweichungsschwelle, die dem Zielabtastpunkt entspricht, ein extremer Ausreißer ist, der der zweiten Sequenz durch die Quartil-Methode zugeordnet ist, und/oder die durchschnittliche Abweichungsschwelle, die dem Zielabtastpunkt entspricht, ein extremer Ausreißer ist, der der dritten Sequenz durch die Quartil-Methode zugeordnet ist.

11. Elektronisches Gerät (100), wobei das elektronische Gerät Folgendes umfasst: einen oder mehrere Prozessoren, einen Speicher (121) und ein Display (171) und
Der Speicher (121) ist mit den einen oder mehreren Prozessoren (110) gekoppelt, der Speicher (121) ist dazu konfiguriert, Computercode zu speichern, der Computercode umfasst Computeranweisungen, und die einen oder mehreren Prozessoren rufen die Computeranweisungen auf, um das elektronische Gerät dazu zu befähigen, das Verfahren gemäß einem der Ansprüche 1 bis 10 auszuführen.

12. Computerlesbares Speichermedium (121), das Anweisungen umfasst, wobei, wenn die Anweisungen auf einem elektronischen Gerät (100) ausgeführt werden, das elektronische Gerät (100) dazu befähigt wird, das Verfahren gemäß einem der Ansprüche 1 bis 10 auszuführen.


**Revendications**

1. Procédé de suppression du bruit dans un signal d'électrocardiographie, le procédé étant exécuté par un dispositif électronique comprenant un processeur, le procédé comprenant :

   déterminer (S601) une première séquence de caractéristiques correspondant à un point d'échantillonnage cible, la première séquence de caractéristiques correspondant au point d'échantillonnage cible comprenant j éléments, les j éléments correspondent, de manière un-à-un, à j points d'échantillonnage dans un signal devant subir une suppression de bruit ; un élément correspondant à n'importe quel point d'échantillonnage parmi les j points d'échantillonnage représente la somme des amplitudes ascendantes ou descendantes de tous les points d'échantillonnage situés entre ce point et un premier point d'échantillonnage ; une amplitude ascendante ou descendante du point d'échantillonnage concerné est égale à la différence entre l'amplitude de ce point et celle du point suivant ; et le premier point d'échantillonnage est un point situé avant ce point, ayant une tendance de variation opposée à celle du point concerné, et étant le plus proche de celui-ci ; et
   moyenner (S602) une amplitude du point d'échantillonnage cible et les amplitudes des m points d'échantillonnage autour de ce point cible, et obtenir une valeur numérique cible dans le cas où les valeurs numériques dans la première séquence de caractéristiques correspondant au point d'échantillonnage cible sont toutes inférieures à un seuil de variation cumulative correspondant au point d'échantillonnage cible, et mettre à jour l'amplitude du point d'échantillonnage cible à la valeur numérique cible ; le seuil de variation cumulative correspondant au point d'échantillonnage cible étant déterminé à l'aide de la première séquence de caractéristiques associée au point d'échantillonnage cible et de v premières séquences de caractéristiques associées à v points d'échantillonnage précédant ce point, où v = 1 et m est égal à trente pour cent de la fréquence d'échantillonnage des points devant subir la suppression du bruit.

2. Le procédé selon la revendication 1, dans lequel
avant la moyenne de l'amplitude du point d'échantillonnage cible et des amplitudes des m points autour du point d'échantillonnage cible, et avant l'obtention d'une valeur numérique cible, le procédé comprend en outre :

   déterminer une première valeur de caractéristique correspondant au point d'échantillonnage cible, la première valeur de caractéristique correspondant au point d'échantillonnage cible représentant une moyenne des valeurs absolues des différences d'amplitude entre chaque paire de points d'échantillonnage adjacents dans une fenêtre temporelle comprenant le point d'échantillonnage cible et comprenant (k+1) points d'échantillonnage ; et
   la moyenne de l'amplitude du point d'échantillonnage cible et des amplitudes des m points autour du point d'échantillonnage cible, et l'obtention d'une valeur numérique cible, comprennent :
   en moyennant l'amplitude du point d'échantillonnage cible et les amplitudes des m points d'échantillonnage autour du point d'échantillonnage cible, et en obtenant la valeur numérique cible dans le cas où les valeurs numériques de la première séquence de caractéristiques correspondant au point d'échantillonnage cible sont toutes inférieures au seuil de variation cumulée correspondant au point d'échantillonnage cible, et où la première valeur de caractéristique correspondant au point d'échantillonnage cible est inférieure à un seuil moyen de variation, le seuil moyen de variation correspondant au point d'échantillonnage cible étant déterminé à partir de la première valeur de caractéristique correspondant au point d'échantillonnage cible et des premières valeurs de

EP 4 442 194 B1

caractéristique des i points d'échantillonnage ; et dans le signal devant subir une suppression de bruit, les i points d'échantillonnage sont les i points d'échantillonnage avant le point d'échantillonnage cible.

3. Le procédé selon la revendication 1 ou 2, le procédé comprenant en outre :

conserver l'amplitude du point d'échantillonnage cible inchangée dans le cas où au moins une valeur numérique de la première séquence de caractéristiques correspondant au point d'échantillonnage cible est supérieure ou égale au seuil de variation cumulée correspondant au point d'échantillonnage cible ; et/ou
conserver l'amplitude du point d'échantillonnage cible inchangée dans le cas où la première valeur de caractéristique correspondant au point d'échantillonnage cible est supérieure ou égale au seuil moyen de variation.

4. Le procédé selon l'une quelconque des revendications 1 à 3, dans lequel, avant la détermination d'une première séquence de caractéristiques correspondant à un point d'échantillonnage cible, le procédé comprend en outre :
effectuer un filtrage passe-haut et un filtrage passe-bas sur un signal initial, et obtenir le signal devant subir une suppression de bruit, le signal initial étant un signal électrique représentant le rythme cardiaque d'un utilisateur et obtenu par un dispositif électronique.

5. Le procédé selon l'une quelconque des revendications 2 à 4, dans lequel, dans la fenêtre temporelle comprenant le point d'échantillonnage cible et les (k+1) points d'échantillonnage, le point d'échantillonnage cible est le dernier point d'échantillonnage de la fenêtre temporelle comprenant les (k+1) points d'échantillonnage ; ou dans la fenêtre temporelle comprenant le point d'échantillonnage cible et les (k+1) points d'échantillonnage, il existe au moins un point d'échantillonnage avant le point d'échantillonnage cible et au moins un point d'échantillonnage après le point d'échantillonnage cible.

6. Le procédé selon l'une quelconque des revendications 1 à 5, dans lequel la détermination d'une première séquence de caractéristiques correspondant à un point d'échantillonnage cible comprend :

prendre le point d'échantillonnage cible comme point de départ, et déterminer une première fenêtre temporelle comprenant (j+1) points d'échantillonnage ;
calculer une différence d'amplitude entre chaque point d'échantillonnage, à l'exception du dernier point d'échantillonnage dans la première fenêtre temporelle, et un point d'échantillonnage suivant le point d'échantillonnage de manière séquentielle, et obtenir j différences ;
réinitialiser les valeurs numériques inférieures à 0 parmi les j différences en 0, et obtenir une première séquence correspondant au point d'échantillonnage cible ; et
effectuer une opération d'accumulation et de reconstruction vers l'avant sur les éléments de la première séquence correspondant au point d'échantillonnage cible de manière séquentielle, obtenir les j éléments, et prendre les j éléments comme la première séquence de caractéristiques correspondant au point d'échantillonnage cible, l'opération d'accumulation et de reconstruction comprenant : garder la valeur d'un élément inchangée si la valeur de l'élément est 0 ; et, si la valeur de l'élément n'est pas nulle, accumuler la valeur de l'élément et la valeur de l'élément précédent jusqu'à rencontrer un élément ayant une valeur de 0, et prendre la valeur obtenue par l'accumulation avant comme valeur après la reconstruction de l'élément.

7. Le procédé selon l'une quelconque des revendications 2 à 6, dans lequel la détermination d'une première valeur de caractéristique correspondant au point d'échantillonnage cible comprend :

déterminer une seconde fenêtre temporelle comprenant (k+1) points d'échantillonnage, dans laquelle, dans la seconde fenêtre temporelle, n points d'échantillonnage existent avant le point d'échantillonnage cible, et (k-n) points d'échantillonnage existent après le point d'échantillonnage cible ; et
calculer une valeur absolue de la différence entre chaque point d'échantillonnage et un point d'échantillonnage précédent dans la seconde fenêtre temporelle de manière séquentielle, obtenir les valeurs absolues des k différences, et prendre la moyenne des valeurs absolues des k différences comme la première valeur de caractéristique correspondant au point d'échantillonnage cible.

8. Le procédé selon l'une quelconque des revendications 1 à 7, dans lequel, après la détermination d'une première séquence de caractéristiques correspondant à un point d'échantillonnage cible, le procédé comprend en outre :

assembler la première séquence de caractéristiques correspondant au point d'échantillonnage cible et les v premières séquences de caractéristiques correspondant aux v points d'échantillonnage, pour obtenir une seconde séquence,
déterminer une valeur aberrante correspondant à la seconde séquence par une méthode des quartiles, et
déterminer cette valeur aberrante comme le seuil de variation cumulative correspondant au point d'échantillonnage cible.

9. Le procédé selon l'une quelconque des revendications 2 à 8, dans lequel, après la détermination d'une première valeur de caractéristique correspondant au point d'échantillonnage cible, le procédé comprend en outre :

construction d'une troisième séquence en utilisant la première valeur de caractéristique correspondant au point d'échantillonnage cible et les i premières valeurs de caractéristiques correspondant aux i points d'échantillonnage,
détermination d'une valeur aberrante correspondant à la troisième séquence selon la méthode des quartiles, et
détermination de la valeur aberrante correspondant à la troisième séquence comme seuil moyen de variation correspondant au point d'échantillonnage cible.

10. Procédé selon l'une quelconque des revendications 8 ou 9, dans lequel le seuil cumulatif de variation correspondant au point d'échantillonnage cible est une valeur aberrante extrême correspondant à la deuxième séquence déterminée selon la méthode des quartiles, et/ou le seuil moyen de variation correspondant au point d'échantillonnage cible est une valeur aberrante extrême correspondant à la troisième séquence déterminée selon la méthode des quartiles.

11. Un dispositif électronique (100), ledit dispositif électronique comprenant : un ou plusieurs processeurs, une mémoire (121) et un affichage (171),
la mémoire (121) est couplée au(x) processeur(s) (110), la mémoire (121) est configurée pour stocker un code de programme informatique, le code de programme informatique comprenant des instructions informatiques, et le(s) processeur(s) exécutent les instructions informatiques afin de permettre au dispositif électronique d'exécuter le procédé selon l'une quelconque des revendications 1 à 10.

12. Un support de stockage informatique lisible (121), comprenant des instructions, lesquelles, lorsqu'elles sont exécutées sur un dispositif électronique (100), permettent audit dispositif électronique (100) d'exécuter le procédé selon l'une quelconque des revendications 1 à 10.

FIG. 1

FIG. 2

FIG. 3

**(A)**

**(B)**

FIG. 4

Electronic device 100

Antenna

Wireless communication module
BT/WLAN/GNSS/NFC/IR/FM
[150]

Loudspeaker
[170A]

Receiver
[170B]

Microphone
[170C]

Audio
Module

[170]

Display [171]

Key 172

Internal memory
[121]

External memory
interface [120]

Processor

[110]

Sensor module [180]

Pressure sensor
[180A]

Gyroscope sensor
[180B]

Acceleration sensor
[180C]

Capacitive proximity
sensor [180D]

Electrocardiography
sensor [180E]

Ambient light sensor
[180F]

Universal serial
bus (USB)
interface [130]

Charging
input

Charging
management
module
[140]

Power
management
module [141]

Battery [142]

FIG. 5

S601

An electronic device determines a first feature sequence corresponding to a target sampling point

S602

In a case that numerical values in a first feature sequence corresponding to the target sampling point are all less than a cumulative variation threshold corresponding to the target sampling point, an electronic device averages an amplitude of the target sampling point and amplitudes of m sampling points around the target sampling point, obtains a target numerical value, and updates the amplitude of the target sampling point to the target numerical value

FIG. 6

FIG. 7

EP 4 442 194 B1

FIG. 8

FIG. 9

| | S1001 |
|---|---|
| An electronic device determines a first feature sequence corresponding to a target sampling point | |

| | S1002 |
|---|---|
| An electronic device determines a first feature value corresponding to the above target sampling point | |

| | S1003 |
|---|---|
| In cases that numerical values in a first feature sequence corresponding to the target sampling point are all less than a cumulative variation threshold corresponding to the target sampling point, and a first feature value corresponding to the target sampling point is less than an average variation threshold, an electronic device averages an amplitude of the target sampling point and amplitudes of m sampling points around the target sampling point, obtains a target numerical value, and updates the amplitude of the target sampling point to the target numerical value | |

FIG. 10

FIG. 11

**EP 4 442 194 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202211202646

- CN 113040784 A